# EUROPEAN PATENT APPLICATION

(11) **EP 4 523 698 A1**
(43) Date of publication of application: **19.03.2025**
(21) Application number: 23802885.6
(22) Date of filing: 09.05.2023
(51) Int. Cl.: A61K 36/736, A61K 36/73, A23L 33/105, A23L 33/00, A23L 21/12, A23L 19/00, A23L 2/02, A61P 9/10

(54) **COMPOSITION OF MALUS NIEDZWETZKYANA DIECK AND PRUNUS CERASIFERA EHRHART AND USE THEREOF IN TERMS OF RESISTING ATHEROSCLEROSIS**

(30) Priority: 13.05.2022 CN 202210521226
(71) Applicant: ZHEJIANG YANGSHENGTANG INSTITUTE OF NATURAL MEDICATION CO., LTD., HANGZHOU, Zhejiang 310024 (CN)
(72) Inventor: SHEN, Yan, Hangzhou, Zhejiang 310024 (CN); LI, Dong, Hangzhou, Zhejiang 310024 (CN); HU, Liu, Hangzhou, Zhejiang 310024 (CN)
(74) Representative: J A Kemp LLP
(86) International application number: PCT/CN2023/092926
(87) International publication number: WO 2023/217115

(57) **Abstract**

A composition of *Malus niedzwetzkyana* Dieck and *Prunus cerasifera* Ehrhart, wherein the weight ratio of *Malus niedzwetzkyana* Dieck to *Prunus cerasifera* Ehrhart is 1:10-10:1. The composition has a synergistic effect in terms of preventing and-or treating atherosclerosis. The product forms of the composition of *Malus niedzwetzkyana* Dieck and *Prunus cerasifera* Ehrhart include a fruit juice, a jam, a fruit powder, oral liquid products, capsules, tablets, etc.

## Description

### Technical field

The invention relates to the field of food and medicine, in particular to composition of *Malus niedzwetzkyana* Dieck and *Prunus cerasifera* Ehrhart and use thereof in terms of resisting atherosclerosis.

### Background art

Atherosclerosis (As) is an inflammatory disease involving arterial wall characterized by the accumulation of lipids and inflammatory cells in large arterial intima. Atherosclerosis can lead to atherosclerotic cardiovascular disease (ASCVD). Clinically diagnosed ASCVD diseases mainly include: acute coronary syndrome, history of myocardial infarction, stable or unstable angina, coronary revascularization (including interventional therapy and bypass surgery), other peripheral arterial diseases or revascularization post-operations, atherosclerosis-derived stroke or transient ischemic attack. At present, ASCVD has become the leading cause of death worldwide and seriously endangers health. The incidence of atherosclerosis in China has a tendency of obvious increase, and is often seen in middle-aged and old people. Research shows that by 2020, 2 billion people worldwide have carotid atherosclerosis. A survey involving 85,000 people in China showed that one-third of the elderly have atherosclerosis.

Clinical drugs for treating atherosclerosis mainly include: (1) lipid-lowering drugs: statins, ezetimibe, PCSK9 inhibitors, and the like; (2) anti-platelet drugs: aspirin, clopidogrel, and the like. The pathogenesis of AS is complex. A large number of researches indicate that low density lipoprotein cholesterol (LDL-C) plays a key role in the occurrence and development of AS, and lipid-lowering drugs are the preferred drugs. In addition, there are also oxidative stress theory and inflammation theory as to the pathogenesis of atherosclerosis. At present, there are some literature reports or clinical trials, such as colchicine and canakinumab related to inflammation theory, probucol, idebenone and the like related to oxidative stress. There are some Chinese patent medicines in clinical treatment for resisting atherosclerosis, such as Compound Danshen Dripping Pills, Qishen Yiqi Dripping Pills, Naoxintong Capsules and the like. Atherosclerosis requires a treatment regimen under the guidance of a physician and requires long-term medication. Different drugs have different degrees of side effects on the human body, such as liver and muscle damage, gastrointestinal reactions and the like, so a more natural and safe way for intervention is needed.

*Malus niedzwetzkyana* Dieck is a plant of Malus Mill of Rosaceae, and the flesh of its fruit is red, which has very high potential utilization value in the aspects of research, processing and utilization of germplasm resources. *Malus niedzwetzkyana* Dieck is in many varieties, greatly differing in the fruit size and color. China, New Zealand, Japan, Switzerland and other countries have carried out the breeding of *Malus niedzwetzkyana* Dieck. Specifically, it includes Hongxun series in Xinjiang, China, Robert Crab in the United States, Ruby Sweet in Japan, Redlove in Switzerland and the like. *Malus niedzwetzkyana* Dieck in Xinjiang plays an important role in the origin of red flesh Malus Mill plant.

*Prunus cerasifera* Ehrhart, commonly called wild plum, is a plant of Prunus of Rosaceae. Wild *Prunus cerasifera* Ehrhart is listed as a national Class II key protected species. It is a rare and endangered original wild fruit tree species resource in the world. It is rarely naturally distributed in the world. The Daxigou and Xiaoxigou of the Ili river valley in Xinjiang are the largest centralized distribution areas of wild *Prunus cerasifera* Ehrhart in the world. The fruit of *Prunus cerasifera* Ehrhart has a long history of consumption in Xinjiang, China. The Kazakh and Mongolian herdsmen living in Daxigou of Tianshan Mountains mainly eat beef and mutton, but few of them suffer from hyperlipidemia and hypertension. This is related to the long-term use habit of herdsmen picking wild *Prunus cerasifera* Ehrhart to make jam or drying the fruit to make tea. *Prunus cerasifera* Ehrhart includes a plurality of varieties, which are mainly classified according to color into black fruits, red fruits, purple fruits, purple black fruits, yellow fruits and the like.

*Malus niedzwetzkyana* Dieck and *Prunus cerasifera* Ehrhart are rich in anthocyanin, polyphenol, flavonoid and other functional components, and have high development value. However, the current research is still limited, mainly focused on chemical components and oxidation resistance, and still in the preliminary research stage.

### Contents of the invention

After a great deal of experimental research, the inventor of the invention finds that a combination of *Malus niedzwetzkyana* Dieck (or *Malus niedzwetzkyana* Dieck extract) and *Prunus cerasifera* Ehrhart (or *Prunus cerasifera* Ehrhart extract) has a synergistic effect in terms of resisting atherosclerosis.

To this end, in a first aspect, the invention provides a composition comprising:
a first component which is *Malus niedzwetzkyana* Dieck or *Malus niedzwetzkyana* Dieck extract; and
a second component which is *Prunus cerasifera* Ehrhart or *Prunus cerasifera* Ehrhart extract.

In some embodiments, the weight ratio of the first component to the second component is 1:10 to 10:1 (such as 1:9-10:1, 1:8-10:1, 1:7-10:1, 1:6-10:1, 1:5-10:1, 1:4-10:1, 1:3-10:1, 1:2-10:1, 1:1-10:1, 1:10-9:1, 1:9-9:1, 1:8-9:1, 1:7-9:1, 1:6-9:1, 1:5-9:1, 1:4-9:1, 1:3-9:1, 1:2-9:1, 1:1-9:1, 1:10-8:1, 1:9-8:1, 1:8-8:1, 1:7-8:1, 1:6-8:1, 1:5-8:1, 1:4-8:1, 1:3-8:1, 1:2-8:1, 1:1-8:1, 1:10-7:1, 1:9-7:1, 1:8-7:1, 1:7-7:1, 1:6-7:1, 1:5-7:1, 1:4-7:1, 1:3-7:1, 1:2-7:1, 1:1-7:1, 1:10-6:1, 1:9-6:1, 1:8-6:1, 1:7-6:1, 1:6-6:1, 1:5-6:1, 1:4-6:1, 1:3-6:1, 1:2-6:1, 1:1-6:1, 1:10-5:1, 1:9-5:1, 1:8-5:1, 1:7-5:1, 1:6-5:1, 1: 5-5:1, 1:4-5:1, 1:3-5:1, 1:2-5:1, 1:1-5:1, 1:10-4:1, 1:9-4:1, 1:8-4:1, 1:7-4:1, 1:6-4:1, 1:5-4:1, 1:4-4:1, 1:3-4:1, 1:2-4:1, 1:1-4:1, 1:10-3:1, 1:9-3:1, 1:8-3:1, 1:7-3:1, 1:6-3:1, 1:5-3:1, 1:4-3:1, 1:3-3:1, 1:2-3:1, 1:1-3:1, 1:10-2:1, 1:9-2:1, 1:8-2:1, 1:7-2:1, 1:6-2:1, 1:5-2:1, 1:4-2:1, 1:3-2:1, 1:2-2:1, 1:1-2:1, 1:10-1:1, 1:9-1:1, 1:8-1:1, 1:7-1:1, 1:6-1:1, 1:5-1:1, 1:4-1:1, 1:3-1:1, 1:2-1:1, 1:10-5:1, 1:10-2:1, 1:10-1:1, 1:5-10:1, 1:5-5:1, 1:5-2:1, 1:5-1:1, 1:2-10:1, 1:2-5:1, 1:2-2:1, 1:2-1:1, 1:1-10:1, 1:1-5:1, 1:1-2:1, e.g., 1:9, 1:8, 1:7, 1:6, 1:5, 1:4, 1:3, 1:2, 1:1, 2:1, 3:1, 41, 51, 6:1, 7:1, 8:1, 9:1).

In some embodiments, the *Malus niedzwetzkyana* Dieck or *Malus niedzwetzkyana* Dieck extract is obtained from/by: fresh fruit, fresh fruit pulping or juice extraction, leaching extraction, ultrasonic extraction, ultra-high pressure extraction, subcritical extraction, macroporous resin separation or other extraction methods.

In some embodiments, the *Prunus cerasifera* Ehrhart or *Prunus cerasifera* Ehrhart extract is obtained from/by: fresh fruit, fresh fruit pulping or juice extraction, leaching extraction, ultrasonic extraction, ultra-high pressure extraction, subcritical extraction, macroporous resin separation or other extraction methods.

In some embodiments, the composition is in the form of fresh fruit, juice, jam, powder (e.g., lyophilized powder), candy (e.g., tableted candy), oral liquid, capsule, tablet, dried fruit, preserved fruit, wine, or pastry. In other words, the product form of the composition can be fresh fruit, juice, jam, powder (e.g., lyophilized powder), candy (e.g., tableted candy), oral liquid, capsule, tablet, dried fruit, preserved fruit, wine, or pastry. That is, the composition can be made into fresh fruit, juice, jam, powder (e.g., lyophilized powder), candy (e.g., tableted candy), oral liquid, capsule, tablet, dried fruit, preserved fruit, wine, or pastry. Among them, the juice, jam, powder (e.g., lyophilized powder), candy (e.g., tableted candy), oral liquid, capsule, tablet can be prepared from extracts obtained by leaching extraction, ultrasonic extraction, ultra-high pressure extraction, subcritical extraction, macroporous resin separation or other extraction methods.

In some embodiments, the composition is in the form of a drug (e.g., traditional Chinese medicine, Chinese patent medicine, natural medicine and the like), ordinary food or functional food or health food. In other words, the composition can be made into a drug (e.g., traditional Chinese medicine, Chinese patent medicine, natural medicine and the like), ordinary food or functional food or health food.

In some embodiments, the *Malus niedzwetzkyana* Dieck refers to a fruit of a plant of Malus Mill of Rosaceae with red flesh.

In some embodiments, the *Malus niedzwetzkyana* Dieck is selected from: Hongxun 1 (Hong Xun 1), Hongxun 3 (Hong Xun 3), Hongxun 4 (Hong Xun 4), Hongxun 6 (Hong Xun 6), Hongxun 8 (Hong Xun 8), Red Love and the like.

In some embodiments, the *Prunus cerasifera* Ehrhart is selected from *Prunus cerasifera* Ehrhart red fruit, *Prunus cerasifera* Ehrhart black fruit, *Prunus cerasifera* Ehrhart purple fruit, *Prunus cerasifera* Ehrhart purple black fruit, *Prunus cerasifera* Ehrhart yellow fruit, and the like.

In a second aspect, the invention provides a combination product comprising:
a first product, said first product being the aforementioned composition;
a second product, said second product being a drug for preventing and/or treating atherosclerotic disease.

In some embodiments, the atherosclerotic disease is selected from atherosclerosis of aorta and its major branches, coronary atherosclerosis, cerebral atherosclerosis, renal atherosclerosis, mesenteric atherosclerosis, extremity atherosclerosis and the like.

In some embodiments, the drug for preventing and/or treating atherosclerotic disease is selected from statins (e.g., rosuvastatin, atorvastatin, simvastatin), ezetimibe, PCSK9 inhibitor, aspirin, clopidogrel, colchicine, connamab, probucol, idebenone and the like.

In some embodiments, the weight ratio of the first product to the second product is 1000:0.001 to 1000:33.4 (e.g., 1000:0.002075, 1000:0.004175, 1000:0.0083, 1000:0.02075, 1000:0.04175, 1000:0.083, 1000:0.08325, 1000:0.167, 1000:0.20875, 1000:0.3125, 1000:0.333, 1000:0.4175, 1000:0.62625, 1000:0.835, 1000:1.25, 1000:1.67, 1000:2.0875, 1000:2.505, 1000:2.875, 1000:4.175, 1000:8.35, or 1000:16.7, or such as 1000:0.002075 to 1000:16.7).

In some embodiments, when the second product is rosuvastatin, the weight ratio of the first product to the second product is 1000:0.01 to 1000:0.34 (e.g., 1000:0.02075, 1000:0.04175, 1000:0.083 or 1000:0.167, or, e.g., 1000:0.02075 to 1000: 0.167).

In some embodiments, when the second product is atorvastatin, the weight ratio of the first product to the second product is 1000:0.02 to 1000:0.67 (e.g., 1000:0.04175, 1000:0.08325, 1000:0.167 or 1000:0.333, or such as 1000:0.04175 to 1000:0.333).

In some embodiments, when the second product is aspirin, the weight ratio of the first product to the second product is 1000:0.10 to 1000:5.01 (e.g., 1000:0.20875, 1000:0.4175, 1000:0.62625, 1000:0.835, 1000:1.67, or 1000:2.505, or, e.g., 1000:0.20875 to 1000:2.505).

In some embodiments, when the second product is clopidogrel, the weight ratio of the first product to the second product is 1000:0.10 to 1000:5.01 (e.g., 1000:0.20875, 1000:0.4175, 1000:0.62625, 1000:0.835, 1000:1.67, or 1000:2.505, or, e.g., 1000:0.20875 to 1000:2.505).

In some embodiments, when the second product is colchicine, the weight ratio of the first product to the second product is 1000:0.001 to 1000:0.166 (e.g., 1000:0.002075, 1000:0.004175, 1000:0.0083, 1000:0.02075 or 1000:0.083, or, e.g., 1000:0.002075 to 1000:0.083).

In some embodiments, when the second product is probucol, the weight ratio of the first product to the second product is 1000:1.0 to 1000:33.4 (e.g., 1000:2.0875, 1000:2.875, 1000:4.175, 1000:8.35, or 1000:16.7, or, e.g., 1000:2.0875 to 1000:16.7).

In a third aspect, the invention provides use of the aforementioned composition in preparation of medicine (e.g., traditional Chinese medicine, Chinese patent medicine, natural medicine and the like), ordinary food or functional food or health food for preventing and/or treating atherosclerotic disease. The invention also provides use of the aforementioned combination product in preparation of medicine (e.g., traditional Chinese medicine, Chinese patent medicine, natural medicine and the like) for preventing and/or treating atherosclerotic disease.

In the invention, the ordinary food refers to finished products and raw materials for human consumption or drinking, as well as articles that are traditionally both food and medicine, but do not include articles for therapeutic purposes.

In the invention, the health food refers to a food which is claimed to have a specific health function or aims to supplement vitamins and minerals, namely a food which is suitable for specific people, has the function of regulating human body, does not aim to treat diseases and does not cause any acute, subacute or chronic harm to human body. The functional food is mainly a name of food capable of improving physical health conditions or reducing diseases in Europe and Japan, which is similar in definition to health food in China.

In the invention, the traditional Chinese medicine is a drug for which the Chinese traditional medicine theory is utilized to guide the collection, processing and preparation, explain the mechanism of action and guide the clinical application. The traditional Chinese medicine is mainly derived from natural medicine and its processed products, including plant medicine, animal medicine, mineral medicine and some chemical and biological products, most of which is plant medicine.

In the invention, the Chinese patent medicine, which is prepared into a Chinese medicinal product in a certain dosage form for preventing and treating diseases according to specific prescription and preparation process under the guidance of the Chinese traditional medicine theory, is a commercialized Chinese medicinal preparation approved by the national drug administration and supervision department.

In the invention, the natural medicine refers to animal medicine, plant medicine, mineral medicine and the like which have been proved to have certain pharmacological activities by the modern medical system. The natural medicine is not equivalent to the traditional Chinese medicine or Chinese herb. The natural medicine refers to natural medicinal substances and preparations thereof used under the guidance of modern medical theory. Its source includes plant, animal and mineral, generally excluding substances derived from genetically modified animal and plant, substances which are subjected to microbial fermentation or chemical modification and the like.

The invention also provides a combination product comprising a first product and a second product packaged separately, the first product being a combination product of *Malus niedzwetzkyana* Dieck and *Prunus cerasifera* Ehrhart, and the second product being an anti-atherosclerotic drug (e.g., lipid-lowering, anti-platelet, anticoagulant, anti-inflammatory, anti-oxidative stress drugs and the like).

In the invention, "individual" includes human or non-human animal. Exemplary human individuals include human individuals (referred to as patients) with a disease (e.g., a disease described herein) or normal individuals. "Non-human animal" in the invention includes all vertebrates, such as non-mammals (e.g., fish, birds, amphibians, reptiles) and mammals, such as non-human primates, livestock and/or domesticated animals (e.g., rats, sheep, dogs, cats, cows, pigs and the like).

In the invention, "*Prunus cerasifera* Ehrhart extract" or "*Malus niedzwetzkyana* Dieck extract" refers to a product formed by subjecting *Prunus cerasifera* Ehrhart or *Malus niedzwetzkyana* Dieck as raw material to a physical and/or chemical extraction and separation process, thereby obtaining and enriching one or more effective components in *Prunus cerasifera* Ehrhart or *Malus niedzwetzkyana* Dieck, which can be used as a raw material or adjuvant of food (such as ordinary food or functional food or health food) or medicine. Exemplary extraction method includes, but is not limited to leaching extraction, ultrasonic extraction, ultra-high pressure extraction, subcritical extraction, macroporous resin separation and the like.

Exemplary methods of preparing *Malus niedzwetzkyana* Dieck and *Prunus cerasifera* Ehrhart extracts are provided below.

### Leaching extraction

In some embodiments, the *Malus niedzwetzkyana* Dieck and *Prunus cerasifera* Ehrhart extracts of the invention are obtained by leaching extraction (e.g., water leaching extraction or alcohol leaching extraction).

In some embodiments, the leaching extraction comprises the steps of:
step 1: removing seeds or cores of *Malus niedzwetzkyana* Dieck and *Prunus cerasifera* Ehrhart respectively, and pulping to obtain pulps directly, or separating juice from pomace; in some embodiments, juice and pomace are separated by filtration using gauze (such as 100-300 mesh gauze, e.g., 200 mesh gauze);
step 2: mixing the pulp or pomace with an extraction medium (such as water or ethanol), adding cellulase, extracting at room temperature or under heating (such as 10-100°C, e.g., 50°C) (such as for 30-100 min, e.g., for 60 min), and filtering to obtain pulp or pomace extracts; in some embodiments, the pulp or pomace is mixed with the extraction medium in a weight ratio of 1: (2-10) (e.g., 1:5); in some embodiments, the ethanol is 20% to 100% (e.g., 20%, 30%, 40%, 50%, 60%, 70%, 80%, 90%, or 100%) ethanol;
step 3: concentrating the pulp extracts or combined solutions of the juices and the pomace extracts to obtain *Malus niedzwetzkyana* Dieck and *Prunus cerasifera* Ehrhart leaching extracts; in some embodiments, the concentration is performed by rotary evaporation; in some embodiments, the rotary evaporation temperature is 30-60°C, e.g., 50°C;
step 4: mixing the *Malus niedzwetzkyana* Dieck and *Prunus cerasifera* Ehrhart extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then extracting.

In some embodiments, the *Malus niedzwetzkyana* Dieck and *Prunus cerasifera* Ehrhart extracts of the invention are obtained by water leaching extraction, which in some embodiments comprises the steps of:
step 1: removing seeds or cores of *Malus niedzwetzkyana* Dieck and *Prunus cerasifera* Ehrhart respectively, and pulping to obtain pulps directly, or filtering by 100-300 mesh gauze to obtain *Malus niedzwetzkyana* Dieck and *Prunus cerasifera* Ehrhart juices, thereby separating juice from pomace; in some embodiments, the gauze has a mesh number of 200 mesh;
step 2: mixing *Malus niedzwetzkyana* Dieck and *Prunus cerasifera* Ehrhart pulps or pomaces with water according to the feed to liquid ratio of 1: (2-10), adding cellulase, extracting at 10-100°C for 30-100 min, and filtering to obtain pulp or pomace extracts; in some embodiments, the feed to liquid ratio is selected to be 1:5; in some embodiments, the leaching extraction temperature is selected to be 50°C; in some embodiments, the extraction time is selected to be 60 min; in some embodiments, the cellulase is added in an amount (based on the weight of the pomace or pulp) ranging from 0.2% to 1% (e.g., 0.5%);
step 3: concentrating the *Malus niedzwetzkyana* Dieck and *Prunus cerasifera* Ehrhart pulp extracts or combined solutions of the juices and the pomace extracts by rotary evaporation at 30-60°C to obtain *Malus niedzwetzkyana* Dieck and *Prunus cerasifera* Ehrhart water leaching extracts; In some embodiments, the rotary evaporation temperature is selected to be 50°C;
step 4: mixing the *Malus niedzwetzkyana* Dieck and *Prunus cerasifera* Ehrhart water leaching extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or first mixing *Malus niedzwetzkyana* Dieck and *Prunus cerasifera* Ehrhart raw materials according to the aforementioned different ratios in step 1 and then extracting.

In some embodiments, the *Malus niedzwetzkyana* Dieck and *Prunus cerasifera* Ehrhart extracts of the invention are obtained by alcohol leaching extraction, which in some embodiments comprises the steps of:
step 1: removing seeds or cores of *Malus niedzwetzkyana* Dieck and *Prunus cerasifera* Ehrhart respectively, and pulping to obtain pulps directly, or filtering by 100-300 mesh gauze to obtain *Malus niedzwetzkyana* Dieck and *Prunus cerasifera* Ehrhart juices, thereby separating juice from pomace; in some embodiments, the gauze has a mesh number of 200 mesh;
step 2: mixing *Malus niedzwetzkyana* Dieck and *Prunus cerasifera* Ehrhart pulps or pomaces with 20-100% (e.g., 20%, 30%, 40%, 50%, 60%, 70%, 80%, 90% or 100%) ethanol according to the feed to liquid ratio of 1: (2-10), adding 0.2%-1% (based on the weight of the pomace or pulp) (e.g., 0.3%, 0.4%, 0.5%, 0.6%, 0.7%, 0.8%, 0.9% or 1%) of cellulase, extracting at 10-100°C for 30-100 min, and filtering to obtain pulp or pomace extracts; in some embodiments, the feed to liquid ratio is selected to be 1:5; in some embodiments, the concentration of the ethanol used is 40%; in some embodiments, the leaching extraction temperature is selected to be 50°C; in some embodiments, the extraction time is selected to be 60 min.
step 3: concentrating the *Malus niedzwetzkyana* Dieck and *Prunus cerasifera* Ehrhart pulp extracts or combined solutions of the juices and the pomace extracts by rotary evaporation at 30-60°C, directly concentrating the pulp extracts, to obtain *Malus niedzwetzkyana* Dieck and *Prunus cerasifera* Ehrhart alcohol leaching extracts; In some embodiments, the rotary evaporation temperature is selected to be 50°C; optionally, the extracts are evaporated till dryness to remove residual ethanol;
step 4: mixing the *Malus niedzwetzkyana* Dieck and *Prunus cerasifera* Ehrhart alcohol leaching extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or first mixing *Malus niedzwetzkyana* Dieck and *Prunus cerasifera* Ehrhart raw materials according to the aforementioned different ratios in step 1 and then extracting.

### Ultrasonic extraction

In some embodiments, the *Malus niedzwetzkyana* Dieck and *Prunus cerasifera* Ehrhart extracts of the invention are obtained by ultrasonic extraction (e.g., ultrasonic water extraction or ultrasonic alcohol extraction).

In some embodiments, the ultrasonic extraction comprises the steps of:
step 1: removing seeds or cores of *Malus niedzwetzkyana* Dieck and *Prunus cerasifera* Ehrhart respectively, and pulping to obtain pulps directly, or separating juice from pomace; in some embodiments, juice and pomace are separated by filtration using gauze (such as 100-300 mesh gauze, e.g., 200 mesh gauze);
step 2: mixing the pulp or pomace with an extraction medium (such as water or ethanol) and cellulase, extracting under heating (such as 30-50°C, e.g., 45°C) (such as for 30-100 min, e.g., for 60 min), treating under ultrasonic conditions (such as 100-500W ultrasound power) (such as for 20-60 min, e.g., for 30 min), and filtering to obtain pulp or pomace extracts; in some embodiments, the cellulase is added in an amount (based on the weight of the pomace or pulp) ranging from 0.2% to 1% (e.g., 0.5%); in some embodiments, the pulp or pomace and the extraction medium are mixed in a weight ratio of 1: (2-10) (e.g., 1:5); in some embodiments, the ethanol is 20-100% (e.g., 20%, 30%, 40%, 50%, 60%, 70%, 80%, 90%, or 100%) ethanol;
step 3: concentrating the *Malus niedzwetzkyana* Dieck and *Prunus cerasifera* Ehrhart pulp extracts or combined solutions of the juices and the pomace extracts to obtain *Malus niedzwetzkyana* Dieck and *Prunus cerasifera* Ehrhart ultrasonic extracts; in some embodiments, the concentration is performed by rotary evaporation; In some embodiments, the rotary evaporation temperature is 30-60°C, e.g., 50°C;
step 4: mixing the *Malus niedzwetzkyana* Dieck and *Prunus cerasifera* Ehrhart ultrasonic extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then extracting.

In some embodiments, the *Malus niedzwetzkyana* Dieck and *Prunus cerasifera* Ehrhart extracts of the invention are obtained by ultrasonic water extraction, which in some embodiments comprises the steps of:
step 1: removing seeds or cores of *Malus niedzwetzkyana* Dieck and *Prunus cerasifera* Ehrhart respectively, and pulping to obtain pulps directly, or filtering by 100-300 mesh gauze to obtain *Malus niedzwetzkyana* Dieck and *Prunus cerasifera* Ehrhart juices, thereby separating juice from pomace; in some embodiments, the gauze has a mesh number of 200 mesh;
step 2: mixing *Malus niedzwetzkyana* Dieck and *Prunus cerasifera* Ehrhart pulps or pomaces with water according to the feed to liquid ratio of 1: (2-10), adding 0.2%-1% (based on the weight of the pomace or pulp) of cellulase, extracting in a water bath at 30-50°C for 30-100 min, treating with 100-500W ultrasound for 20-60 min, and filtering to obtain pulp or pomace extracts; in some embodiments, the feed to liquid ratio is selected to be 1:5; in some embodiments, the cellulase is added in an amount of 0.5%; in some embodiments, the water bath temperature is 45°C; in some embodiments, the extraction time is selected to be 60 min; in some embodiments, the ultrasound power is 500W; in some embodiments, the ultrasonic treatment time is 30 min;
step 3: concentrating the *Malus niedzwetzkyana* Dieck and *Prunus cerasifera* Ehrhart pulp extracts or combined solutions of the juices and the pomace extracts by rotary evaporation at 30-60°C to obtain *Malus niedzwetzkyana* Dieck and *Prunus cerasifera* Ehrhart ultrasonic water extracts; in some embodiments, the rotary evaporation temperature is selected to be 50°C;
step 4: mixing the *Malus niedzwetzkyana* Dieck and *Prunus cerasifera* Ehrhart ultrasonic water extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or first mixing *Malus niedzwetzkyana* Dieck and *Prunus cerasifera* Ehrhart raw materials according to the aforementioned different ratios in step 1 and then extracting.

In some embodiments, the *Malus niedzwetzkyana* Dieck and *Prunus cerasifera* Ehrhart extracts of the invention are obtained by ultrasonic alcohol extraction, which in some embodiments comprises the steps of:
step 1: removing seeds or cores of *Malus niedzwetzkyana* Dieck and *Prunus cerasifera* Ehrhart respectively, and pulping to obtain pulps directly, or filtering by 100-300 mesh gauze to obtain *Malus niedzwetzkyana* Dieck and *Prunus cerasifera* Ehrhart juices, thereby separating juice from pomace; in some embodiments, the gauze has a mesh number of 200 mesh;
step 2: mixing *Malus niedzwetzkyana* Dieck and *Prunus cerasifera* Ehrhart pulps or pomaces with 20%-100% ethanol according to the feed to liquid ratio of 1: (2-10), adding 0.2%-1% (based on the weight of the pomace or pulp) of cellulase, extracting in a water bath at 30-50°C for 30-100 min, treating with 100-500W ultrasound for 20-60 min, and filtering to obtain pulp or pomace extracts; in some embodiments, the feed to liquid ratio is selected to be 1:5; in some embodiments, the concentration of the ethanol used is 40%; in some embodiments, the cellulase is added in an amount of 0.5%; in some embodiments, the water bath temperature is 45°C; in some embodiments, the extraction time is selected to be 60 min; in some embodiments, the ultrasound power is 500W; in some embodiments, the ultrasonic treatment time is 30 min;
step 3: concentrating the *Malus niedzwetzkyana* Dieck and *Prunus cerasifera* Ehrhart pulp extracts or combined solutions of the juices and the pomace extracts by rotary evaporation at 30-60°C to obtain *Malus niedzwetzkyana* Dieck and *Prunus cerasifera* Ehrhart ultrasonic alcohol extracts; in some embodiments, the rotary evaporation temperature is selected to be 50°C; optionally, the extracts are evaporated till dryness to remove residual ethanol;
step 4: mixing the *Malus niedzwetzkyana* Dieck and *Prunus cerasifera* Ehrhart ultrasonic alcohol extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or first mixing *Malus niedzwetzkyana* Dieck and *Prunus cerasifera* Ehrhart raw materials according to the aforementioned different ratios in step 1 and then extracting.

### Ultra-high pressure extraction

In some embodiments, *Malus niedzwetzkyana* Dieck and *Prunus cerasifera* Ehrhart extracts of the invention are obtained by ultra-high pressure extraction (e.g., ultra-high pressure water extraction or ultra-high pressure alcohol extraction). As used herein, "ultra-high pressure" refers to pressures in excess of 100 MPa (equivalent to 1000 atmospheres).

In some embodiments, the ultra-high pressure extraction comprises the steps of:
step 1: removing seeds or cores of *Malus niedzwetzkyana* Dieck and *Prunus cerasifera* Ehrhart respectively, and pulping to obtain pulps directly, or separating juice from pomace; in some embodiments, juice and pomace are separated by filtration using gauze (such as 100-300 mesh gauze, e.g., 200 mesh gauze);
step 2: mixing the pulp or pomace with an extraction medium (such as water or ethanol), treating under an ultra-high pressure condition (such as 200-600 MPa, e.g., 400 MPa) (such as for 3-10 min, e.g., for 5 min), and filtering to obtain pulp or pomace extracts; in some embodiments, the pulp or pomace is mixed with the extraction medium in a weight ratio of 1: (2-10) (e.g., 1: 5); in some embodiments, the ethanol is 50%-95% ethanol;
step 3: concentrating the *Malus niedzwetzkyana* Dieck and *Prunus cerasifera* Ehrhart pulp extracts or combined solutions of the juices and the pomace extracts to obtain *Malus niedzwetzkyana* Dieck and *Prunus cerasifera* Ehrhart ultra-high pressure extracts; in some embodiments, the concentration is performed by rotary evaporation; in some embodiments, the rotary evaporation temperature is 30-60°C, e.g., 50°C;
step 4: mixing the *Malus niedzwetzkyana* Dieck and *Prunus cerasifera* Ehrhart ultra-high pressure extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then extracting.

In some embodiments, the *Malus niedzwetzkyana* Dieck and *Prunus cerasifera* Ehrhart extracts of the invention are obtained by ultra-high pressure water extraction, which in some embodiments comprises the steps of:
step 1: removing seeds or cores of *Malus niedzwetzkyana* Dieck and *Prunus cerasifera* Ehrhart respectively, and pulping to obtain pulps directly, or filtering by 100-300 mesh gauze to obtain *Malus niedzwetzkyana* Dieck and *Prunus cerasifera* Ehrhart juices, thereby separating juice from pomace; in some embodiments, the gauze has a mesh number of 200 mesh;
step 2: mixing *Malus niedzwetzkyana* Dieck and *Prunus cerasifera* Ehrhart pulps or pomaces with water according to the feed to liquid ratio of 1: (2-10), treating under an ultra-high pressure of 200-600 MPa for 3-10 min, and filtering to obtain pulp or pomace extracts; in some embodiments, the feed to liquid ratio is selected to be 1:5; in some embodiments, the ultra-high pressure is 400 MPa; in some embodiments, the ultra-high pressure treatment time is selected to be 5 min;
step 3: concentrating the *Malus niedzwetzkyana* Dieck and *Prunus cerasifera* Ehrhart pulp extracts or combined solutions of the juices and the pomace extracts by rotary evaporation at 30-60°C to obtain *Malus niedzwetzkyana* Dieck and *Prunus cerasifera* Ehrhart ultra-high pressure water extracts; in some embodiments, the rotary evaporation temperature is selected to be 50°C;
step 4: mixing the *Malus niedzwetzkyana* Dieck and *Prunus cerasifera* Ehrhart ultra-high pressure water extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or first mixing *Malus niedzwetzkyana* Dieck and *Prunus cerasifera* Ehrhart raw materials according to the aforementioned different ratios in step 1 and then extracting.

In some embodiments, the *Malus niedzwetzkyana* Dieck and *Prunus cerasifera* Ehrhart extracts of the invention are obtained by ultra-high pressure alcohol extraction, which in some embodiments comprises the steps of:
step 1: removing seeds or cores of *Malus niedzwetzkyana* Dieck and *Prunus cerasifera* Ehrhart respectively, and pulping to obtain pulps directly, or filtering by 100-300 mesh gauze to obtain *Malus niedzwetzkyana* Dieck and *Prunus cerasifera* Ehrhart juices, thereby separating juice from pomace; in some embodiments, the gauze has a mesh number of 200 mesh;
Step 2: mixing *Malus niedzwetzkyana* Dieck and *Prunus cerasifera* Ehrhart pulps or pomaces with 20%-100% (e.g., 20%, 30%, 40%, 50%, 60%, 70%, 80%, 90% or 100%) ethanol according to the feed to liquid ratio of 1: (2-10), treating under an ultra-high pressure of 200-600 MPa for 3-10 min, and filtering to obtain pulp or pomace extracts; in some embodiments, the feed to liquid ratio is selected to be 1:5; in some embodiments, the ultra-high pressure is 400 MPa; in some embodiments, the ultra-high pressure treatment time is selected to be 5 min; in some embodiments, the concentration of the ethanol used is 40%;
step 3: concentrating the *Malus niedzwetzkyana* Dieck and *Prunus cerasifera* Ehrhart pulp extracts or combined solutions of the juices and the pomace extracts by rotary evaporation at 30-60°C to obtain *Malus niedzwetzkyana* Dieck and *Prunus cerasifera* Ehrhart ultra-high pressure alcohol extracts; in some embodiments, the rotary evaporation temperature is selected to be 50°C; optionally, the extracts are evaporated till dryness to remove residual ethanol;
step 4: mixing the *Malus niedzwetzkyana* Dieck and *Prunus cerasifera* Ehrhart ultra-high pressure alcohol extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or first mixing *Malus niedzwetzkyana* Dieck and *Prunus cerasifera* Ehrhart raw materials according to the aforementioned different ratios in step 1 and then extracting.

### Subcritical extraction

In some embodiments, *Malus niedzwetzkyana* Dieck and *Prunus cerasifera* Ehrhart extracts are obtained by subcritical extraction (e.g., subcritical water extraction or subcritical alcohol extraction).

In some embodiments, the subcritical extraction comprises the steps of:
step 1: removing seeds or cores of *Malus niedzwetzkyana* Dieck and *Prunus cerasifera* Ehrhart respectively, and pulping to obtain pulps directly, or separating juice from pomace; in some embodiments, juice and pomace are separated by filtration using gauze (such as 100-300 mesh gauze, e.g., 200 mesh gauze);
step 2: mixing the pulp or pomace with an extraction medium (such as water or ethanol), performing subcritical extraction treatment (such as for 10-50 min, e.g., for 30 min), and filtering to obtain pulp or pomace extracts; in some embodiments, the pulp or pomace is mixed with the extraction medium in a weight ratio of 1: (2-10) (e.g., 1: 5); in some embodiments, the ethanol is 50%-95% ethanol; in some embodiments, the subcritical extraction treatment temperature is 100-150°C (e.g., 110°C);
step 3: concentrating the pulp extracts or combined solutions of the juices and the pomace extracts to obtain *Malus niedzwetzkyana* Dieck and *Prunus cerasifera* Ehrhart subcritical extracts; in some embodiments, the concentration is performed by rotary evaporation; in some embodiments, the rotary evaporation temperature is 30-60°C, e.g., 50°C;
step 4: mixing the *Malus niedzwetzkyana* Dieck and *Prunus cerasifera* Ehrhart subcritical extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then extracting.

In some embodiments, *Malus niedzwetzkyana* Dieck and *Prunus cerasifera* Ehrhart extracts of the invention are obtained by subcritical water extraction, which In some embodiments comprises the steps of:
step 1: removing seeds or cores of *Malus niedzwetzkyana* Dieck and *Prunus cerasifera* Ehrhart respectively, and pulping to obtain pulps directly, or filtering by 100-300 mesh gauze to obtain *Malus niedzwetzkyana* Dieck and *Prunus cerasifera* Ehrhart juices, thereby separating juice from pomace; In some embodiments, the gauze has a mesh number of 200 mesh;
step 2: mixing *Malus niedzwetzkyana* Dieck and *Prunus cerasifera* Ehrhart pulps or pomaces with water according to the feed to liquid ratio of 1: (2-10), performing subcritical extraction for 10-50 min at the treatment temperature of 100-150°C, and filtering to obtain pulp or pomace extracts; In some embodiments, the feed to liquid ratio is selected to be 1:5; In some embodiments, the treatment temperature is 110°C; In some embodiments, the treatment time is 30 min;
step 3: concentrating the *Malus niedzwetzkyana* Dieck and *Prunus cerasifera* Ehrhart pulp extracts or combined solutions of the juices and the pomace extracts by rotary evaporation at 30-60°C to obtain *Malus niedzwetzkyana* Dieck and *Prunus cerasifera* Ehrhart subcritical water extracts; In some embodiments, the rotary evaporation temperature is selected to be 50°C;
step 4: mixing the *Malus niedzwetzkyana* Dieck and *Prunus cerasifera* Ehrhart subcritical water extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or first mixing *Malus niedzwetzkyana* Dieck and *Prunus cerasifera* Ehrhart raw materials according to the aforementioned different ratios in step 1 and then extracting.

In some embodiments, the *Malus niedzwetzkyana* Dieck and *Prunus cerasifera* Ehrhart extracts of the invention are obtained by subcritical alcohol extraction, which in some embodiments comprises the steps of:
step 1: removing seeds or cores of *Malus niedzwetzkyana* Dieck and *Prunus cerasifera* Ehrhart respectively, and pulping to obtain pulps directly, or filtering by 100-300 mesh gauze to obtain *Malus niedzwetzkyana* Dieck and *Prunus cerasifera* Ehrhart juices, thereby separating juice from pomace; in some embodiments, the gauze has a mesh number of 200 mesh;
step 2: mixing *Malus niedzwetzkyana* Dieck and *Prunus cerasifera* Ehrhart pulps or pomaces with 20%-100% (e.g., 20%, 30%, 40%, 50%, 60%, 70%, 80%, 90% or 100%) ethanol according to the feed to liquid ratio of 1: (2-10), performing subcritical extraction for 10-50 min at the treatment temperature of 100-150°C, and filtering to obtain pulp or pomace extracts; in some embodiments, the feed to liquid ratio is selected to be 1:5; in some embodiments, the treatment temperature is 110°C; in some embodiments, the treatment time is 30 min;
step 3: concentrating the *Malus niedzwetzkyana* Dieck and *Prunus cerasifera* Ehrhart pulp extracts or combined solutions of the juices and the pomace extracts by rotary evaporation at 30-60°C to obtain *Malus niedzwetzkyana* Dieck and *Prunus cerasifera* Ehrhart subcritical alcohol extracts; in some embodiments, the rotary evaporation temperature is selected to be 50°C; optionally, the extracts are evaporated till dryness to remove residual ethanol;
step 4: mixing the *Malus niedzwetzkyana* Dieck and *Prunus cerasifera* Ehrhart subcritical alcohol extracts according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or first mixing *Malus niedzwetzkyana* Dieck and *Prunus cerasifera* Ehrhart raw materials according to the aforementioned different ratios in step 1 and then extracting.

### Macroporous resin separation

In some embodiments, *Malus niedzwetzkyana* Dieck and *Prunus cerasifera* Ehrhart extracts are obtained by macroporous resin separation.

In some embodiments, the macroporous resin separation comprises the steps of:
step 1: obtaining *Malus niedzwetzkyana* Dieck and *Malus niedzwetzkyana* Dieck extracts by the aforementioned different extraction methods (leaching extraction, ultrasonic extraction, ultra-high pressure extraction, subcritical extraction and the like); or obtaining *Malus niedzwetzkyana* Dieck and *Malus niedzwetzkyana* Dieck pulps by directly pulping;
step 2: mixing the *Malus niedzwetzkyana* Dieck and *Malus niedzwetzkyana* Dieck extracts or pulps with 95%-100% ethanol according to the ratio of 1: (2-10) (by weight) (e.g., 1:2, 1:3, 1:4, 1:5, 1:6, 1:7, 1:8, 1:9, or 1:10), for 1-5 hr (e.g., 3 hr), then filtering to obtain alcohol precipitation juices and precipitates, and subjecting the alcohol precipitation juices to rotary evaporation at 30-60°C (e.g., 30°C, 40°C, 50°C or 60°C) to remove ethanol, thereby obtaining clear alcohol precipitation juices; in some embodiments, the *Malus niedzwetzkyana* Dieck and *Malus niedzwetzkyana* Dieck extracts or pulps can also be subjected to enzymolysis clarification treatment with the addition of pectinase to obtain clear enzymolysis juices, wherein the enzymolysis temperature is 20-60°C (e.g., 20°C, 30°C, 40°C, 50°C or 60°C), the enzymolysis time is 30-240 min (e.g., 30 min, 60 min, 90 min, 120 min, 150 min, 180 min, 210 min or 240 min), and the amount of the pectinase added is 100-1000 ppm (e.g., 100 ppm, 200 ppm, 300 ppm, 400 ppm, 500 ppm, 600 ppm, 700 ppm, 800 ppm, 900 ppm or 1000 ppm);
step 3: adsorbing the clear alcohol precipitation juices or clear enzymolysis juices with macroporous adsorbent resin, sequentially performing gradient elution with water and 20%-100% ethanol (e.g., several ones selected from 20% ethanol, 40% ethanol, 60% ethanol, 80% ethanol, and 100% ethanol), while collecting eluates of each gradient respectively; in some embodiments, the elution rate is 1-5 BV/h (1-5 column volumes);
step 4: subjecting the eluates to rotary evaporation at 30-60°C (e.g., 30°C, 40°C, 50°C or 60°C) to remove ethanol, thereby obtaining different macroporous resin separation samples.

*Malus niedzwetzkyana* Dieck and *Prunus cerasifera* Ehrhart can be extracted separately to obtain extracts, which are then mixed at certain ratios to obtain compositions, or *Malus niedzwetzkyana* Dieck and *Prunus cerasifera* Ehrhart raw materials can be first mixed according to different ratios, and then extracted to obtain compositions. As mentioned before, the product form of the composition of the invention can be fresh fruit, juice, jam, powder (e.g., lyophilized powder), candy (e.g., tableted candy), oral liquid, capsule, tablet, dried fruit, preserved fruit, wine, or pastry. Among them, the juice, jam, powder (e.g., lyophilized powder), candy (e.g., tableted candy), oral liquid, capsule, tablet can be prepared from extracts obtained by leaching extraction, ultrasonic extraction, ultra-high pressure extraction, subcritical extraction, macroporous resin separation or other extraction methods. In addition, the product form of the composition of the invention can be a drug, ordinary food or functional food or health food. In some embodiments, in the product forms including juice, jam, powder (e.g., lyophilized powder), candy (e.g., tableted candy), oral liquid, capsule, tablet, dried fruit, preserved fruit, wine, or pastry, the composition of the invention can be used as a main raw material, for example, accounting for 30 wt% or more, 40 wt% or more, 50 wt% or more, 60 wt% or more, 70 wt% or more, 80 wt% or more, 90 wt% or more, or 100 wt% of the total raw materials; optionally, the product can also contain other components such as water, sweetening substances, or other kinds of fruits or extracts thereof.

Exemplary methods of preparing *Malus niedzwetzkyana* Dieck and *Prunus cerasifera* Ehrhart products (e.g., fresh fruit, juice, jam, powder, tableted candy, oral liquid, capsule) are provided below.

### Fresh fruit

In some embodiments, *Malus niedzwetzkyana* Dieck and *Prunus cerasifera* Ehrhart fresh fruits can be obtained by a method comprising the steps of:
step 1: collecting *Malus niedzwetzkyana* Dieck and *Prunus cerasifera* Ehrhart from 1 month before ripening to complete ripening, preferably, collecting the fruits in sunny days;
step 2: picking to remove rotten or poor-colored fruits;
step 3: storing in refrigeration after picking;
step 4: combining *Malus niedzwetzkyana* Dieck and *Prunus cerasifera* Ehrhart fresh fruits according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1).

The *Malus niedzwetzkyana* Dieck and *Prunus cerasifera* Ehrhart fresh fruits obtained can be directly eaten.

### Juice

In some embodiments, *Malus niedzwetzkyana* Dieck and *Prunus cerasifera* Ehrhart juices can be obtained by a method comprising the steps of:
step 1: removing seeds or cores of *Malus niedzwetzkyana* Dieck and *Prunus cerasifera* Ehrhart respectively, and then pulping;
step 2: filtering the pulps (for example, using 100-300 mesh gauze) to obtain *Malus niedzwetzkyana* Dieck and *Prunus cerasifera* Ehrhart cloudy juices; in some embodiments, the gauze has a mesh number of 200 mesh;
step 3: mixing the *Malus niedzwetzkyana* Dieck and *Prunus cerasifera* Ehrhart cloudy juices according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1 to prepare compositions, or first mixing *Malus niedzwetzkyana* Dieck and *Prunus cerasifera* Ehrhart raw materials according to the aforementioned different ratios in step 1 and then pulping to prepare the juices.

In some embodiments, *Malus niedzwetzkyana* Dieck and *Prunus cerasifera* Ehrhart juices can also be obtained by a method comprising the steps of:
step 1: removing seeds or cores of *Malus niedzwetzkyana* Dieck and *Prunus cerasifera* Ehrhart respectively, and then pulping;
step 2: filtering the pulps (for example, using 100-300 mesh gauze), adding pectinase for enzymolysis and filtering, to obtain *Malus niedzwetzkyana* Dieck and *Prunus cerasifera* Ehrhart clear juices; in some embodiments, the gauze has a mesh number of 200 mesh;
step 3: mixing the *Malus niedzwetzkyana* Dieck and *Prunus cerasifera* Ehrhart clear juices according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1 to prepare compositions, or first mixing *Malus niedzwetzkyana* Dieck and *Prunus cerasifera* Ehrhart raw materials according to the aforementioned different ratios in step 1 and then pulping to prepare the juices.

In some embodiments, *Malus niedzwetzkyana* Dieck and *Prunus cerasifera* Ehrhart juices can also be prepared using, as raw material, the extracts obtained by the aforementioned leaching extraction, ultrasonic extraction, ultra-high pressure extraction, subcritical extraction, macroporous resin separation or other extraction methods.

### Jam

In some embodiments, *Malus niedzwetzkyana* Dieck and *Prunus cerasifera* Ehrhart jams can be obtained by a method comprising the steps of:
step 1: removing seeds or cores of *Malus niedzwetzkyana* Dieck and *Prunus cerasifera* Ehrhart respectively, and then pulping;
step 2: boiling the pulps in water, concentrating under heating to obtain *Malus niedzwetzkyana* Dieck and *Prunus cerasifera* Ehrhart jams;
step 3: mixing the *Malus niedzwetzkyana* Dieck and *Prunus cerasifera* Ehrhart jams according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then pulping to prepare the jams.

In some embodiments, *Malus niedzwetzkyana* Dieck and *Prunus cerasifera* Ehrhart jams can also be prepared using, as raw material, the extracts or juices obtained by the aforementioned leaching extraction, ultrasonic extraction, ultra-high pressure extraction, subcritical extraction, macroporous resin separation or other extraction methods.

### Powder

In some embodiments, *Malus niedzwetzkyana* Dieck and *Prunus cerasifera* Ehrhart powders can be obtained by a method comprising the steps of:
step 1: removing seeds or cores of *Malus niedzwetzkyana* Dieck and *Prunus cerasifera* Ehrhart respectively, and then pulping;
step 2: lyophilizing the pulps to obtain pulp lyophilized powders;
step 3: mixing the *Malus niedzwetzkyana* Dieck and *Prunus cerasifera* Ehrhart lyophilizedpowders according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then pulping to prepare the lyophilized powders.

In some embodiments, *Malus niedzwetzkyana* Dieck and *Prunus cerasifera* Ehrhart powders can also be obtained by a method comprising the steps of:
step 1: removing seeds or cores of *Malus niedzwetzkyana* Dieck and *Prunus cerasifera* Ehrhart respectively, and then pulping;
step 2: filtering the pulps (for example, using 100-300 mesh gauze), to obtain *Malus niedzwetzkyana* Dieck and *Prunus cerasifera* Ehrhart juices; in some embodiments, the gauze has a mesh number of 200 mesh;
step 3: lyophilizing the juices to obtain juice lyophilized powders;
step 4: mixing the *Malus niedzwetzkyana* Dieck and *Prunus cerasifera* Ehrhart lyophilized powders according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then pulping to prepare the lyophilizedpowders.

In some embodiments, *Malus niedzwetzkyana* Dieck and *Prunus cerasifera* Ehrhart powders can also be obtained using a beltdrier, with reference to the above steps.

In some embodiments, *Malus niedzwetzkyana* Dieck and *Prunus cerasifera* Ehrhart powders can also be prepared using, as raw material, the extracts obtained by the aforementioned leaching extraction, ultrasonic extraction, ultra-high pressure extraction, subcritical extraction, macroporous resin separation or other extraction methods, through lyophilization or belt drying.

### Tableted candy

In some embodiments, *Malus niedzwetzkyana* Dieck and *Prunus cerasifera* Ehrhart tableted candies can be obtained by a method comprising the steps of:
step 1: removing seeds or cores of *Malus niedzwetzkyana* Dieck and *Prunus cerasifera* Ehrhart respectively, and then pulping;
step 2: lyophilizing or belt drying the pulps to obtain powders;
step 3: tableting the powders and adjuvants to obtain tableted candies;
step 4: mixing the *Malus niedzwetzkyana* Dieck and *Prunus cerasifera* Ehrhart tableted candies according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1 to prepare compositions, or first mixing *Malus niedzwetzkyana* Dieck and *Prunus cerasifera* Ehrhart raw materials according to the aforementioned different ratios in step 1 and then pulping to prepare the tableted candies.

In some embodiments, *Malus niedzwetzkyana* Dieck and *Prunus cerasifera* Ehrhart tableted candies can also be obtained by a method comprising the steps of:
step 1: removing seeds or cores of *Malus niedzwetzkyana* Dieck and *Prunus cerasifera* Ehrhart respectively, and then pulping;
step 2: filtering the pulps (for example, using 100-300 mesh gauze), to obtain *Malus niedzwetzkyana* Dieck and *Prunus cerasifera* Ehrhart juices; in some embodiments, the gauze has a mesh number of 200 mesh;
step 3: lyophilizing or belt drying the juices to obtain powders;
step 4: tableting the powders and adjuvants to obtain tableted candies;
step 5: mixing the *Malus niedzwetzkyana* Dieck and *Prunus cerasifera* Ehrhart tableted candies according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1 to prepare compositions, or first mixing *Malus niedzwetzkyana* Dieck and *Prunus cerasifera* Ehrhart raw materials according to the aforementioned different ratios in step 1 and then pulping to prepare the tableted candies.

In some embodiments, *Malus niedzwetzkyana* Dieck and *Prunus cerasifera* Ehrhart tableted candies can also be prepared using, as raw material, the extracts obtained by the aforementioned leaching extraction, ultrasonic extraction, ultra-high pressure extraction, subcritical extraction, macroporous resin separation or other extraction methods.

### Oral liquid

In some embodiments, *Malus niedzwetzkyana* Dieck and *Prunus cerasifera* Ehrhart oral liquids can be obtained by a method comprising the steps of:
step 1: preparing *Malus niedzwetzkyana* Dieck and *Prunus cerasifera* Ehrhart extracts (e.g., preparing them from fresh fruits by pulping or juicing, leaching extraction, ultrasonic extraction, ultra-high pressure extraction, subcritical extraction, macroporous resin separation, according to the steps described above) according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1);
step 2: sterilizing the *Malus niedzwetzkyana* Dieck and *Prunus cerasifera* Ehrhart extracts to obtain *Malus niedzwetzkyana* Dieck and *Prunus cerasifera* Ehrhart oral liquids.

In some embodiments, *Malus niedzwetzkyana* Dieck and *Prunus cerasifera* Ehrhart oral liquids can also be obtained by a method comprising the steps of:
step 1: preparing *Malus niedzwetzkyana* Dieck and *Prunus cerasifera* Ehrhart extracts (e.g., preparing them from fresh fruits by pulping or juicing, leaching extraction, ultrasonic extraction, ultra-high pressure extraction, subcritical extraction, macroporous resin separation, according to the steps described above) according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1);
step 2: adding pectinase for enzymolysis, and filtering to obtain clear juices;
step 3: sterilizing the *Malus niedzwetzkyana* Dieck and *Prunus cerasifera* Ehrhart clear juices to obtain *Malus niedzwetzkyana* Dieck and *Prunus cerasifera* Ehrhart oral liquids.

### Capsule

In some embodiments, *Malus niedzwetzkyana* Dieck and *Prunus cerasifera* Ehrhart capsules can be obtained by a method comprising the steps of:
step 1: removing seeds or cores of *Malus niedzwetzkyana* Dieck and *Prunus cerasifera* Ehrhart respectively, and then pulping;
step 2: lyophilizing or belt drying the pulps to obtain pulp lyophilized powders;
step 3: mixing the *Malus niedzwetzkyana* Dieck and *Prunus cerasifera* Ehrhart powders according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then pulping to prepare the powders;
step 4: filling the powders into capsule shells, to obtain capsules.

In some embodiments, *Malus niedzwetzkyana* Dieck and *Prunus cerasifera* Ehrhart capsules can also be obtained by a method comprising the steps of:
step 1: removing seeds or cores of *Malus niedzwetzkyana* Dieck and *Prunus cerasifera* Ehrhart respectively, and then pulping;
step 2: filtering the pulps (for example, using 100-300 mesh gauze), to obtain *Malus niedzwetzkyana* Dieck and *Prunus cerasifera* Ehrhart juices; in some embodiments, the gauze has a mesh number of 200 mesh;
step 3: lyophilizing or belt drying the juices to obtain powders;
step 4: mixing the *Malus niedzwetzkyana* Dieck and *Prunus cerasifera* Ehrhart powders according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1 and 10:1) to prepare compositions, or first mixing *Malus niedzwetzkyana* Dieck and *Prunus cerasifera* Ehrhart raw materials according to the aforementioned different ratios in step 1 and then pulping to prepare the powders;
step 5: filling the powders into capsule shells, to obtain capsules.

In some embodiments, *Malus niedzwetzkyana* Dieck and *Prunus cerasifera* Ehrhart capsules can also be prepared using, as raw material, the extracts obtained by the aforementioned leaching extraction, ultrasonic extraction, ultra-high pressure extraction, subcritical extraction, macroporous resin separation or other extraction methods.

### Advantageous effects

In the invention, natural *Malus niedzwetzkyana* Dieck and *Prunus cerasifera* Ehrhart are utilized. The prepared composition of *Malus niedzwetzkyana* Dieck and *Prunus cerasifera* Ehrhart has good effect in terms of resisting atherosclerosis. *Malus niedzwetzkyana* Dieck and *Prunus cerasifera* Ehrhart used in combination have better effect than that of any of the two used alone, and achieve a synergistic effect. In addition, the further use of the composition in combination with a drug has better effect than that of the composition or the drug used alone, and achieves a synergistic effect.

### Detailed Description of Embodiments

Embodiments of the invention will be described in detail below with reference to the examples, but it will be understood by those skilled in the art that the following examples are only illustrative of the invention and should not be construed as limiting the scope of the invention.

### Example 1:

*Malus niedzwetzkyana* Dieck and *Prunus cerasifera* Ehrhart products described in this example refer to *Malus niedzwetzkyana* Dieck and *Prunus cerasifera* Ehrhart fresh fruits.

*Malus niedzwetzkyana* Dieck (Hongxun 1) and *Prunus cerasifera* Ehrhart were collected from 1 month before ripening to complete ripening, preferably in sunny days, while picking to remove rotten or poor-colored fruits, and then the fruits were stored in refrigeration. The obtained *Malus niedzwetzkyana* Dieck and *Prunus cerasifera* Ehrhart fresh fruits could be combined according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) for direct eating.

### Example 2:

*Malus niedzwetzkyana* Dieck and *Prunus cerasifera* Ehrhart products described in this example refer to *Malus niedzwetzkyana* Dieck and *Prunus cerasifera* Ehrhart cloudy juices.

The collected *Malus niedzwetzkyana* Dieck (Hongxun 1) and *Prunus cerasifera* Ehrhart were pulped after removing seeds or cores, and the pulps were filtered by 200 mesh gauze to obtain *Malus niedzwetzkyana* Dieck and *Prunus cerasifera* Ehrhart cloudy juices. The *Malus niedzwetzkyana* Dieck and *Prunus cerasifera* Ehrhart cloudy juices were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then pulped to obtain the juices.

### Example 3:

*Malus niedzwetzkyana* Dieck and *Prunus cerasifera* Ehrhart products described in this example refer to *Malus niedzwetzkyana* Dieck and *Prunus cerasifera* Ehrhart clear juices.

**The** collected *Malus niedzwetzkyana* Dieck (Hongxun 1) and *Prunus cerasifera* Ehrhart were pulped after removing seeds or cores, and the pulps were filtered by 200 mesh gauze to obtain *Malus niedzwetzkyana* Dieck and *Prunus cerasifera* Ehrhart cloudy juices. Pectinase was added for enzymolysis, followed by filtering, to obtain clear juices. The *Malus niedzwetzkyana* Dieck and *Prunus cerasifera* Ehrhart clear juices were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then pulped to obtain the juices.

### Example 4:

*Malus niedzwetzkyana* Dieck and *Prunus cerasifera* Ehrhart products described in this example refer to *Malus niedzwetzkyana* Dieck and *Prunus cerasifera* Ehrhart jams.

*Malus niedzwetzkyana* Dieck (Hongxun 1) and *Prunus cerasifera* Ehrhart were pulped after removing seeds or cores, and boiled in water under heating to obtain relatively thick jams. The *Malus niedzwetzkyana* Dieck and *Prunus cerasifera* Ehrhart jams were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then pulped to obtain the jams.

### Example 5:

*Malus niedzwetzkyana* Dieck and *Prunus cerasifera* Ehrhart products described in this example refer to *Malus niedzwetzkyana* Dieck and *Prunus cerasifera* Ehrhart powders.

*Malus niedzwetzkyana* Dieck (Hongxun 1) and *Prunus cerasifera* Ehrhart were pulped after removing seeds or cores, and lyophilized to obtain pulp lyophilized powders. The *Malus niedzwetzkyana* Dieck and *Prunus cerasifera* Ehrhart lyophilized powders were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then pulped to obtain the powders.

### Example 6:

*Malus niedzwetzkyana* Dieck and *Prunus cerasifera* Ehrhart products described in this example refer to *Malus niedzwetzkyana* Dieck and *Prunus cerasifera* Ehrhart tableted candies.

*Malus niedzwetzkyana* Dieck (Hongxun 1) and *Prunus cerasifera* Ehrhart were pulped after removing seeds or cores, and lyophilized to obtain pulp lyophilized powders. The pulp lyophilized powders and adjuvants were tableted to obtain tableted candies. The *Malus niedzwetzkyana* Dieck and *Prunus cerasifera* Ehrhart tableted candies were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then pulped to obtain the tableted candies.

### Example 7:

*Malus niedzwetzkyana* Dieck and *Prunus cerasifera* Ehrhart products described in this example refer to *Malus niedzwetzkyana* Dieck and *Prunus cerasifera* Ehrhart water leaching extract oral liquids.
*(1) Malus niedzwetzkyana* Dieck (Hongxun 1) and *Prunus cerasifera* Ehrhart were pulped after removing seeds or cores, to obtain *Malus niedzwetzkyana* Dieck and *Prunus cerasifera* Ehrhart pulps.
(2) The *Malus niedzwetzkyana* Dieck and *Prunus cerasifera* Ehrhart pulps were mixed with water according to the feed to liquid ratio of 1:5, cellulase was added, and water leaching extraction was performed at 50°C for 60 min, followed by filtering to obtain pulp extracts.
(3) The extracts were concentrated by rotary evaporation at 50°C, to obtain *Malus niedzwetzkyana* Dieck and *Prunus cerasifera* Ehrhart water leaching extracts.
(4) The *Malus niedzwetzkyana* Dieck and *Prunus cerasifera* Ehrhart extracts were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions.
(5) The compositions of *Malus niedzwetzkyana* Dieck and *Prunus cerasifera* Ehrhart extracts were sterilized to obtain the oral liquids.

### Example 8:

*Malus niedzwetzkyana* Dieck and *Prunus cerasifera* Ehrhart products described in this example refer to *Malus niedzwetzkyana* Dieck (Hongxun 1) and *Prunus cerasifera* Ehrhart alcohol leaching extract oral liquids.

The treatment was carried out with a reference to the steps (1) - (5) of Example 7, wherein the pulps were extracted with 20%, 40%, 60% or 100% ethanol according to the feed to liquid ratio of 1:5 respectively. The alcohol extracts were evaporated till dryness to remove residual ethanol.

### Example 9:

*Malus niedzwetzkyana* Dieck and *Prunus cerasifera* Ehrhart products described in this example refer to *Malus niedzwetzkyana* Dieck (Hongxun 3) and *Prunus cerasifera* Ehrhart water leaching extract oral liquids. The preparation steps were the same as those of Example 7.

### Example 10:

*Malus niedzwetzkyana* Dieck and *Prunus cerasifera* Ehrhart products described in this example refer to *Malus niedzwetzkyana* Dieck (Hongxun 6) and *Prunus cerasifera* Ehrhart water leaching extract oral liquids. The preparation steps were the same as those of Example 7.

### Example 11:

*Malus niedzwetzkyana* Dieck and *Prunus cerasifera* Ehrhart products described in this example refer to *Malus niedzwetzkyana* Dieck and *Prunus cerasifera* Ehrhart ultrasonic water extract oral liquids.
*(1) Malus niedzwetzkyana* Dieck (Hongxun 1) and *Prunus cerasifera* Ehrhart were pulped after removing seeds or cores, to obtain *Malus niedzwetzkyana* Dieck and *Prunus cerasifera* Ehrhart pulps.
(2) The *Malus niedzwetzkyana* Dieck and *Prunus cerasifera* Ehrhart pulps were mixed with water according to the feed to liquid ratio of 1:5, 0.5% (based on the weight of the pulp) of cellulase was added, the pulps were extracted in water bath at 45°C for 60 min, and treatment was performed with 500W ultrasound for 30 min, followed by filtering to obtain pulp extracts.
(3) The extracts were concentrated by rotary evaporation at 50°C, to obtain *Malus niedzwetzkyana* Dieck and *Prunus cerasifera* Ehrhart ultrasonic water extracts.
(4) The *Malus niedzwetzkyana* Dieck and *Prunus cerasifera* Ehrhart extracts obtained by extraction were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions.
(5) The compositions of *Malus niedzwetzkyana* Dieck and *Prunus cerasifera* Ehrhart extracts were sterilized to obtain the oral liquids.

### Example 12:

*Malus niedzwetzkyana* Dieck and *Prunus cerasifera* Ehrhart products described in this example refer to *Malus niedzwetzkyana* Dieck (Hongxun 6) and *Prunus cerasifera* Ehrhart ultrasonic water extract oral liquids. The preparation steps were the same as those of Example 10.

### Example 13:

*Malus niedzwetzkyana* Dieck and *Prunus cerasifera* Ehrhart products described in this example refer to *Malus niedzwetzkyana* Dieck (Hongxun 1) and *Prunus cerasifera* Ehrhart ultrasonic alcohol extract oral liquids.

The treatment was carried out with a reference to the steps (1) - (5) of Example 10, wherein the pulps were extracted with 20%, 40%, 60% or 100% ethanol according to the feed to liquid ratio of 1:5 respectively. The alcohol extracts were evaporated till dryness to remove residual ethanol.

### Example 14:

*Malus niedzwetzkyana* Dieck and *Prunus cerasifera* Ehrhart products described in this example refer to *Malus niedzwetzkyana* Dieck and *Prunus cerasifera* Ehrhart ultra-high pressure water extract oral liquids.
*(1) Malus niedzwetzkyana* Dieck (Hongxun 1) and *Prunus cerasifera* Ehrhart were pulped after removing seeds or cores, to obtain *Malus niedzwetzkyana* Dieck and *Prunus cerasifera* Ehrhart pulps.
(2) The *Malus niedzwetzkyana* Dieck and *Prunus cerasifera* Ehrhart pulps were mixed with water according to the feed to liquid ratio of 1:5, and treated under an ultra-high pressure of 400 MPa for 5 min, followed by filtering to obtain pulp extracts.
(3) The extracts were concentrated by rotary evaporation at 50°C, to obtain *Malus niedzwetzkyana* Dieck and *Prunus cerasifera* Ehrhart ultra-high pressure water extracts.
(4) The *Malus niedzwetzkyana* Dieck and *Prunus cerasifera* Ehrhart extracts were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions.
(5) The compositions of *Malus niedzwetzkyana* Dieck and *Prunus cerasifera* Ehrhart extracts were sterilized to obtain the oral liquids.

### Example 15:

*Malus niedzwetzkyana* Dieck and *Prunus cerasifera* Ehrhart products described in this example refer to *Malus niedzwetzkyana* Dieck (Hongxun 6) and *Prunus cerasifera* Ehrhart ultra-high pressure water extract oral liquids. The preparation steps were the same as those of Example 13.

### Example 16:

*Malus niedzwetzkyana* Dieck and *Prunus cerasifera* Ehrhart products described in this example refer to *Malus niedzwetzkyana* Dieck (Hongxun 1) and *Prunus cerasifera* Ehrhart ultra-high pressure alcohol extract oral liquids.

The treatment was carried out with a reference to the steps (1) - (5) of Example 13, wherein the pulps were extracted with 50% ethanol according to the feed to liquid ratio of 1:5. The alcohol extracts were evaporated till dryness to remove residual ethanol.

### Example 17:

*Malus niedzwetzkyana* Dieck and *Prunus cerasifera* Ehrhart products described in this example refer to *Malus niedzwetzkyana* Dieck and *Prunus cerasifera* Ehrhart subcritical water extract oral liquids.
*(1) Malus niedzwetzkyana* Dieck (Hongxun 1) and *Prunus cerasifera* Ehrhart were pulped after removing seeds or cores, to obtain *Malus niedzwetzkyana* Dieck and *Prunus cerasifera* Ehrhart pulps.
(2) The *Malus niedzwetzkyana* Dieck and *Prunus cerasifera* Ehrhart pulps were mixed with water according to the feed to liquid ratio of 1:5, and subjected to subcritical extraction at 110°C for 30 min, followed by filtering to obtain pulp extracts.
(3) The extracts were concentrated by rotary evaporation at 50°C, to obtain *Malus niedzwetzkyana* Dieck and *Prunus cerasifera* Ehrhart subcritical water extracts.
(4) The *Malus niedzwetzkyana* Dieck and *Prunus cerasifera* Ehrhart extracts were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions.
(5) The compositions of *Malus niedzwetzkyana* Dieck and *Prunus cerasifera* Ehrhart extracts were sterilized to obtain the oral liquids.

### Example 18:

*Malus niedzwetzkyana* Dieck and *Prunus cerasifera* Ehrhart products described in this example refer to *Malus niedzwetzkyana* Dieck and *Prunus cerasifera* Ehrhart jams.

*Malus niedzwetzkyana* Dieck (Hongxun 1) and *Prunus cerasifera* Ehrhart water leaching extract oral liquids were first prepared with a reference to Example 7, and then boiled in water under heating to obtain relatively thick jams, with or without the addition of adjuvants. The *Malus niedzwetzkyana* Dieck and *Prunus cerasifera* Ehrhart jams were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then prepared into the jams.

### Example 19:

*Malus niedzwetzkyana* Dieck and *Prunus cerasifera* Ehrhart products described in this example refer to *Malus niedzwetzkyana* Dieck and *Prunus cerasifera* Ehrhart powders.

*Malus niedzwetzkyana* Dieck (Hongxun 1) and *Prunus cerasifera* Ehrhart water leaching extract oral liquids were first prepared with a reference to Example 7, and then lyophilized to obtain lyophilized powders, with or without the addition of adjuvants. The *Malus niedzwetzkyana* Dieck and *Prunus cerasifera* Ehrhart lyophilized powders were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then prepared into the powders.

### Example 20:

*Malus niedzwetzkyana* Dieck and *Prunus cerasifera* Ehrhart products described in this example refer to *Malus niedzwetzkyana* Dieck and *Prunus cerasifera* Ehrhart jams.

*Malus niedzwetzkyana* Dieck (Hongxun 1) and *Prunus cerasifera* Ehrhart ultrasonic extract oral liquids were first prepared with a reference to Example 11, and then boiled in water under heating to obtain relatively thick jams, with or without the addition of adjuvants. The *Malus niedzwetzkyana* Dieck and *Prunus cerasifera* Ehrhart jams were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then prepared into the jams.

### Example 21:

*Malus niedzwetzkyana* Dieck and *Prunus cerasifera* Ehrhart products described in this example refer to *Malus niedzwetzkyana* Dieck and *Prunus cerasifera* Ehrhart powders.

*Malus niedzwetzkyana* Dieck (Hongxun 1) and *Prunus cerasifera* Ehrhart ultrasonic extract oral liquids were first prepared with a reference to Example 11, and then belt dried to obtain powders, with or without the addition of adjuvants. The *Malus niedzwetzkyana* Dieck and *Prunus cerasifera* Ehrhart powders were mixed according to different ratios (by weight) (1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1) to prepare compositions, or *Malus niedzwetzkyana* Dieck and *Prunus cerasifera* Ehrhart raw materials were first mixed according to the aforementioned different ratios, and then prepared into the powders.

### Example 22:

*Malus niedzwetzkyana* Dieck and *Prunus cerasifera* Ehrhart products described in this example refer to *Malus niedzwetzkyana* Dieck and *Prunus cerasifera* Ehrhart clear alcohol precipitation juices.

*Malus niedzwetzkyana* Dieck (Hongxun 1) and *Prunus cerasifera* Ehrhart water leaching extract concentrates were first prepared with a reference to Example 7. The concentrates were mixed with 95% ethanol according to the weight ratio of 1:2 for 3 hr, followed by filtering to obtain clear alcohol precipitation juices, and removing ethanol by rotary evaporation, to obtain a composition of *Malus niedzwetzkyana* Dieck and *Prunus cerasifera* Ehrhart clear alcohol precipitation juices.

### Example 23:

*Malus niedzwetzkyana* Dieck and *Prunus cerasifera* Ehrhart products described in this example refer to *Malus niedzwetzkyana* Dieck and *Prunus cerasifera* Ehrhart macroporous resin separation products.

*Malus niedzwetzkyana* Dieck (Hongxun 1) and *Prunus cerasifera* Ehrhart clear alcohol precipitation juices were first prepared with a reference to Example 22. The clear alcohol precipitation juices were adsorbed with macroporous adsorbent resin, and subjected to gradient elution with water and 20%, 40%, 60%, and 100% ethanol sequentially at an elution rate of 3 BV/h (3 column volumes), while the eluates of each gradient were collected respectively. The eluates were subjected to rotary evaporation at 30-60°C to remove ethanol, thereby obtaining different macroporous resin separation samples.

Evaluation on effects of the above example products in terms of resisting atherosclerosis:
Experimental example 1: Evaluation on effects of compositions of *Malus niedzwetzkyana* Dieck and *Prunus cerasifera* Ehrhart in different ratios for preventing atherosclerosis in mice

### (1) Test method

Wild type C57 mice were used as a normal control group, rosuvastatin was used as a positive drug, and the efficacy of the composition samples in preventing atherosclerosis was investigated in a high-fat diet induced ApoE-/-mice atherosclerosis model. An atherosclerosis model (namely, model group) was established after 12 weeks of modeling, and a control group (namely normal group) was fed with a common feed for 12 weeks. The sample administration group was administered with the test samples at the same time of modeling, the normal group and the model group were administered with equal doses of distilled water, the formation of aortic atherosclerotic plaque in the mice was observed after continuous administration for 12 weeks, and the plaque area was measured after dissection to calculate the plaque percentage.

### (2) Test results

The test results are shown in Table 1. In the atherosclerosis prevention model, *Malus niedzwetzkyana* Dieck and *Prunus cerasifera* Ehrhart were extracted by water leaching according to Example 7 to obtain *Malus niedzwetzkyana* Dieck water leaching extract and *Prunus cerasifera* Ehrhart water leaching extract. The *Malus niedzwetzkyana* Dieck extract and the *Prunus cerasifera* Ehrhart extract had certain effects when used alone at a dose of 40 g/kg, and had more significant effects when used in combination in a ratio ranging from 1:10 to 10:1 (the dose of the composition was 40 g/kg) compared with the model group. The compositions could obviously reduce the formation of plaque in the artery wall compared with the model group, being effective for preventing atherosclerosis.

The q value was calculated according to the Jin's formula reported in literature: q = E(A + B)/(EA + EB - EA•EB)

Wherein E(A + B) is the inhibition rate of the two drugs used in combination, EA and EB are the respective inhibition rates of the drugs used alone, q < 1 indicates that the two drugs used in combination have an antagonistic effect, and q ≥ 1 indicates that the two drugs used in combination have a synergistic effect.

As could be seen from Table 1, *Malus niedzwetzkyana* Dieck and *Prunus cerasifera* Ehrhart used in combination had a q value of 1 or more, i.e., the effect thereof was significantly better than that of the two used alone. The compositions had a synergistic effect in preventing atherosclerosis.

**Table 1 Effects of compositions of Malus niedzwetzkyana Dieck and Prunus cerasifera Ehrhart in different ratios for preventing atherosclerosis in mice**

| Group | Plaque area % | Inhibition rate compared to the model % | q |
|---|---|---|---|
| Model | 33.02% | / | / |
| Positive drug (rosuvastain) 1.67 mg/kg | 20.93%** | 36.61% | / |
| *Malus niedzwetzkyana* Dieck 40 g/kg | 23.21%* | 29.71% | / |
| *Prunus cerasifera* Ehrhart 40 g/kg | 25.25%* | 23.53% | / |
| *Malus niedzwetzkyana* Dieck: *Prunus cerasifera* Ehrhart 5:1 40 g/kg | 17.59%** | 46.73% | 1.01 |
| *Malus niedzwetzkyana* Dieck: *Prunus cerasifera* Ehrhart 1:1 40 g/kg | 15.64%** | 52.63% | 1.14 |
| *Malus niedzwetzkyana* Dieck: *Prunus cerasifera* Ehrhart 1:5 40 g/kg | 16.92%** | 48.76% | 1.05 |

| | | | |
|---|---|---|---|
| The significance in the above table was in comparison with the model group; * p< 0.05, ** p<0.01, *** p<0.001 | | | |

Experimental example 2: Evaluation on effects of compositions of *Malus niedzwetzkyana* Dieck and *Prunus cerasifera* Ehrhart in different ratios for treating atherosclerosis in mice

### (1) Test method

Wild type C57 mice were used as a normal control group, rosuvastatin was used as a positive drug, and the efficacy (i.e., treating effect) of the composition samples in resisting atherosclerosis was investigated in a high-fat diet induced ApoE-/-mice atherosclerosis model. An atherosclerosis model was established after 17 weeks of modeling, and a control group was fed with a common feed for 17 weeks. The sample administration group was continuously administered with the test samples for 4 weeks after 17 weeks of modeling, and thereafter the formation of aortic atherosclerotic plaque in the mice was observed, and the plaque area was measured after dissection to calculate the plaque percentage.

### (2) Test results

The test results are shown in Table 2. In the atherosclerosis treatment model, *Malus niedzwetzkyana* Dieck and *Prunus cerasifera* Ehrhart were extracted by water leaching according to Example 7 to obtain *Malus niedzwetzkyana* Dieck water leaching extract and *Prunus cerasifera* Ehrhart water leaching extract. The *Malus niedzwetzkyana* Dieck extract and the *Prunus cerasifera* Ehrhart extract had certain effects when used alone at a dose of 40 g/kg, and had more significant effects when used in combination in a ratio ranging from 1:10 to 10:1 (the dose of the composition was 40 g/kg) compared with the model group. The compositions could obviously reduce the plaque area in the artery wall, being effective for treating atherosclerosis.

The q value was calculated with reference to the Jin's formula of Experimental example 1.

As could be seen from Table 2, *Malus niedzwetzkyana* Dieck and *Prunus cerasifera* Ehrhart used in combination had a q value of 1 or more, i.e., the effect thereof was significantly better than that of the two used alone. The compositions had a synergistic effect in treating atherosclerosis.

**Table 2 Effects of compositions of Malus niedzwetzkyana Dieck and Prunus cerasifera Ehrhart in different ratios for treating atherosclerosis in mice**

| Group | Plaque area % | Inhibition rate compared to the model % | q |
|---|---|---|---|
| Model | 42.57% | / | / |
| Positive drug (rosuvastain) 1.67mg/kg | 26.10%* | 38.69% | / |
| *Malus niedzwetzkyana* Dieck 40 g/kg | 31.05%* | 27.06% | / |
| *Prunus cerasifera* Ehrhart 40 g/kg | 30.23%* | 28.99% | / |
| *Malus niedzwetzkyana* Dieck: *Prunus cerasifera* Ehrhart 5:1 40 g/kg | 21.26%** | 50.06% | 1.04 |
| *Malus niedzwetzkyana* Dieck: *Prunus cerasifera* Ehrhart 2:1 40 g/kg | 20.98%** | 50.72% | 1.05 |
| *Malus niedzwetzkyana* Dieck: *Prunus cerasifera* Ehrhart 1:2 40 g/kg | 20.88%** | 50.95% | 1.06 |
| *Malus niedzwetzkyana* Dieck: *Prunus cerasifera* Ehrhart 1:5 40 g/kg | 20.84%** | 51.05% | 1.06 |

| | | | |
|---|---|---|---|
| The significance in the above table was in comparison with the model group; * p< 0.05, ** p<0.01, *** p<0.001 | | | |

Experimental example 3: Evaluation on effects of composition of *Malus niedzwetzkyana* Dieck and *Prunus cerasifera* Ehrhart at different doses for preventing atherosclerosis in mice

### (1) Test method

Wild type C57 mice were used as a normal control group, rosuvastatin was used as a positive drug, and the efficacy of the composition sample in preventing atherosclerosis was investigated in a high-fat diet induced ApoE-/-mice atherosclerosis model. An atherosclerosis model (namely, model group) was established after 12 weeks of modeling, and a control group (namely normal group) was fed with a common feed for 12 weeks. The sample administration group was administered with the test sample at the same time of modeling, the normal group and the model group were administered with equal doses of distilled water, the formation of aortic atherosclerotic plaque in the mice was observed after continuous administration for 12 weeks, and the plaque area was measured after dissection to calculate the plaque percentage.

### (2) Test results

The test results are shown in Table 3. In the atherosclerosis prevention model, the effects of composition of *Malus niedzwetzkyana* Dieck and *Prunus cerasifera* Ehrhart at different doses were compared. *Malus niedzwetzkyana* Dieck and *Prunus cerasifera* Ehrhart were extracted by water leaching according to Example 7 to obtain *Malus niedzwetzkyana* Dieck water leaching extract and *Prunus cerasifera* Ehrhart water leaching extract. The *Malus niedzwetzkyana* Dieck extract and the *Prunus cerasifera* Ehrhart extract had weak effects when used alone at doses of 20 g/kg and 10 g/kg respectively, and had more significant effects when used in combination (the doses of the composition were 20 g/kg and 10 g/kg respectively) compared with the model group. The compositions could obviously reduce the formation of plaque in the artery wall, being effective for preventing atherosclerosis.

The q value was calculated with reference to the Jin's formula of Experimental example 1.

As could be seen from Table 3, *Malus niedzwetzkyana* Dieck and *Prunus cerasifera* Ehrhart used in combination had a q value of 1 or more, i.e., the effect thereof was superior to that of the two used alone. The compositions had a synergistic effect. It could be seen that the compositions of *Malus niedzwetzkyana* Dieck and *Prunus cerasifera* Ehrhart at different doses could achieve a synergistic effect in terms of preventing atherosclerosis in mice.

**Table 3 Effects of composition of Malus niedzwetzkyana Dieck and Prunus cerasifera Ehrhart at different doses for preventing atherosclerosis in mice**

| Group | Plaque area % | Inhibition rate compared to the model % | q |
|---|---|---|---|
| Model | 35.77% | / | / |
| Positive drug (rosuvastain) 1.67 mg/kg | 24.82% | 30.61% | / |
| *Malus niedzwetzkyana* Dieck 20g /kg | 27.37% | 23.48% | / |
| *Prunus cerasifera* Ehrhart 20 g/kg | 28.88% | 19.26% | / |
| *Malus niedzwetzkyana* Dieck: *Prunus cerasifera* Ehrhart 1:2 20 g/kg | 20.95% | 41.43% | 1.08 |
| *Malus niedzwetzkyana* Dieck: *Prunus cerasifera* Ehrhart 2:1 20 g/kg | 22.03% | 38.41% | 1.01 |
| *Malus niedzwetzkyana* Dieck 10 g/kg | 31.92% | 10.76% | / |
| *Prunus cerasifera* Ehrhart 10g/kg | 31.40% | 12.22% | / |
| *Malus niedzwetzkyana* Dieck: *Prunus cerasifera* Ehrhart 1:2 10 g/kg | 26.85% | 24.94% | 1.15 |
| *Malus niedzwetzkyana* Dieck: *Prunus cerasifera* Ehrhart 2:1 10 g/kg | 25.63% | 28.35% | 1.31 |

| | | | |
|---|---|---|---|
| The significance in the above table was in comparison with the model group; * p< 0.05, ** p<0.01, *** p<0.001 | | | |

Experimental example 4: Evaluation on effects of composition of *Malus niedzwetzkyana* Dieck and *Prunus cerasifera* Ehrhart at different doses for treating atherosclerosis in mice

### (1) Test method

Wild type C57 mice were used as a normal control group, rosuvastatin was used as a positive drug, and the efficacy (i.e., treating effect) of the composition samples in resisting atherosclerosis was investigated in a high-fat diet induced ApoE-/-mice atherosclerosis model. An atherosclerosis model was established after 17 weeks of modeling, and a control group was fed with a common feed for 17 weeks. The sample administration group was continuously administered with the test samples for 4 weeks after 17 weeks of modeling, and thereafter the formation of aortic atherosclerotic plaque in the mice was observed, and the plaque area was measured after dissection to calculate the plaque percentage.

### (2) Test results

The test results are shown in Table 4. In the atherosclerosis treatment model, the effects of composition of *Malus niedzwetzkyana* Dieck and *Prunus cerasifera* Ehrhart at different doses were compared. *Malus niedzwetzkyana* Dieck and *Prunus cerasifera* Ehrhart were extracted by water leaching according to Example 7 to obtain *Malus niedzwetzkyana* Dieck water leaching extract and *Prunus cerasifera* Ehrhart water leaching extract. The *Malus niedzwetzkyana* Dieck extract and the *Prunus cerasifera* Ehrhart extract had weak effects when used alone at doses of 20 g/kg and 10 g/kg respectively, and had more significant effects when used in combination (the doses of the composition were 20 g/kg and 10 g/kg respectively) compared with the model group. The compositions could obviously reduce the plaque area in the artery wall.

The q value was calculated with reference to the Jin's formula of Experimental example 1.

As could be seen from Table 4, *Malus niedzwetzkyana* Dieck and *Prunus cerasifera* Ehrhart used in combination had a q value of 1 or more, i.e., the effect thereof was superior to that of the two used alone. The compositions had a synergistic effect. It could be seen that the compositions of *Malus niedzwetzkyana* Dieck and *Prunus cerasifera* Ehrhart at different doses could achieve a synergistic effect in terms of treating atherosclerosis in mice.

**Table 4 Effects of composition of Malus niedzwetzkyana Dieck and Prunus cerasifera Ehrhart at different doses for treating atherosclerosis in mice**

| Group | Plaque area % | Inhibition rate compared to the model % | q |
|---|---|---|---|
| Model | 39.66% | / | / |
| Positive drug (rosuvastain) 1.67 mg/kg | 25.87%** | 34.77% | / |
| *Malus niedzwetzkyana* Dieck 20 g/kg | 32.89%* | 17.07% | / |
| *Prunus cerasifera* Ehrhart 20 g/kg | 34.08%* | 14.07% | / |
| *Malus niedzwetzkyana* Dieck: *Prunus cerasifera* Ehrhart 1:2 20 g/kg | 27.95%* | 29.53% | 1.03 |
| *Malus niedzwetzkyana* Dieck: *Prunus cerasifera* Ehrhart 2:1 20 g/kg | 28.13%* | 29.07% | 1.01 |
| *Malus niedzwetzkyana* Dieck 10 g/kg | 35.92% | 9.43% | / |
| *Prunus cerasifera* Ehrhart 10 g/kg | 35.40% | 10.74% | / |
| *Malus niedzwetzkyana* Dieck: *Prunus cerasifera* Ehrhart 1:2 10 g/kg | 31.85%* | 19.69% | 1.03 |
| *Malus niedzwetzkyana* Dieck: *Prunus cerasifera* Ehrhart 2:1 10 g/kg | 30.52%* | 23.05% | 1.20 |

| | | | |
|---|---|---|---|
| The significance in the above table was in comparison with the model group; * p< 0.05, ** p<0.01, *** p<0.001 | | | |

Experimental example 5: Evaluation on effects of composition in combination with statin drugs for treating atherosclerosis in mice

### (1) Test method

Wild type C57 mice were used as a normal control group, rosuvastatin and atorvastatin were used as positive drugs, and the efficacy (i.e., treating effect) of the composition mixed samples in resisting atherosclerosis was investigated in a high-fat diet induced ApoE-/-mice atherosclerosis model. An atherosclerosis model was established after 17 weeks of modeling, and a control group was fed with a common feed for 17 weeks. The sample administration group was continuously administered with the test samples for 4 weeks after 17 weeks of modeling, and thereafter the formation of aortic atherosclerotic plaque in the mice was observed, and the plaque area was measured after dissection to calculate the plaque percentage.

### (2) Test results

The test results are shown in Table 5. The composition was obtained by extraction according to Example 7. In the atherosclerosis treatment model, the composition of *Malus niedzwetzkyana* Dieck and *Prunus cerasifera* Ehrhart at doses in the range of 40 g/kg to 10 g/kg in combination with high/low doses of positive drugs (rosuvastatin or atorvastatin) could obviously reduce the plaque area in the artery wall compared with the model group, being effective for treating atherosclerosis. The combined use of the composition and the drugs had a synergistic effect compared with the use of them alone (the calculation was made with reference to Experimental example 1).

**Table 5 Effects of composition of Malus niedzwetzkyana Dieck and Prunus cerasifera Ehrhart in combination with statin drugs for treating atherosclerosis in mice**

| Group | Plaque area % | Inhibition rate compared to the model % |
|---|---|---|
| Model | 40.61% | / |
| Positive drug (rosuvastain) 1.67 mg/kg | 27.86%* | 31.40% |
| Positive drug (rosuvastain) 0.83 mg/kg | 33.72%* | 16.97% |
| Positive drug (atorvastatin) 3.33 mg/kg | 29.05%* | 28.47% |
| Positive drug (atorvastatin) 1.67 mg/kg | 32.99%* | 18.76% |
| *Malus niedzwetzkyana* Dieck: *Prunus cerasifera* Ehrhart 1:2 40 g/kg | 21.68%** | 46.61% |
| *Malus niedzwetzkyana* Dieck: *Prunus cerasifera* Ehrhart 1:2 10 g/kg | 31.57%* | 22.26% |
| *Malus niedzwetzkyana* Dieck: *Prunus cerasifera* Ehrhart 1:2 40 g/kg + rosuvastain 1.67 mg/kg | 14.85%** | 63.43% |
| *Malus niedzwetzkyana* Dieck: *Prunus cerasifera* Ehrhart 1:2 40 g/kg + rosuvastain 0.83 mg/kg | 17.39%** | 57.18% |
| *Malus niedzwetzkyana* Dieck: *Prunus cerasifera* Ehrhart 1:2 10 g/kg + rosuvastain 1.67 mg/kg | 21.01%** | 48.26% |
| *Malus niedzwetzkyana* Dieck: *Prunus cerasifera* Ehrhart 1:2 10 g/kg + rosuvastain 0.83 mg/kg | 25.60%** | 36.96% |
| *Malus niedzwetzkyana* Dieck: *Prunus cerasifera* Ehrhart 1:2 40 g/kg + atorvastatin 3.33 mg/kg | 15.62%** | 61.54% |
| *Malus niedzwetzkyana* Dieck: *Prunus cerasifera* Ehrhart 1:2 40 g/kg + atorvastatin | 17.51%** | 56.88% |
| 1.67 mg/kg | | |

| | | |
|---|---|---|
| The significance in the above table was in comparison with the model group; * p< 0.05, ** p<0.01, *** p<0.001 | | |

Experimental example 6: Evaluation on effects of composition in combination with other drugs for treating atherosclerosis in mice

### (1) Test method

Wild type C57 mice were used as a normal control group, aspirin, clopidogrel, colchicine and probucol were used as positive drugs, and the efficacy (i.e., treating effect) of the composition mixed samples in resisting atherosclerosis was investigated in a high-fat diet induced ApoE-/-mice atherosclerosis model. An atherosclerosis model was established after 17 weeks of modeling, and a control group was fed with a common feed for 17 weeks. The sample administration group was continuously administered with the test samples for 4 weeks after 17 weeks of modeling, and thereafter the formation of aortic atherosclerotic plaque in the mice was observed, and the plaque area was measured after dissection to calculate the plaque percentage.

### (2) Test results

The test results are shown in Table 6. The effects of the combinations of different positive drugs and the composition in terms of resisting atherosclerosis were compared. The composition was obtained by extraction according to Example 7. In the atherosclerosis treatment model, the composition of *Malus niedzwetzkyana* Dieck and *Prunus cerasifera* Ehrhart at doses in the range of 40g/kg to 10g/kg in combination with positive drugs (aspirin, clopidogrel, colchicine and probucol) could obviously reduce the plaque area in the artery wall compared with the model group, being effective for treating atherosclerosis. The combined use of the composition and the drugs had a synergistic effect compared with the use of them alone (the calculation was made with reference to Experimental example 1).

**Table 6 Effects of composition of Malus niedzwetzkyana Dieck and Prunus cerasifera Ehrhart in combination with other drugs for treating atherosclerosis in mice**

| Group | Plaque area % | Inhibition rate compared to the model % |
|---|---|---|
| Model | 45.14% | / |
| Positive drug (aspirin) 16.7 mg/kg | 29.91%* | 33.74% |
| Positive drug (clopidogrel) 12.5 mg/kg | 30.35%* | 32.76% |
| Positive drug (colchicine) 0.167 mg/kg | 33.80%* | 25.12% |
| Positive drug (probucol) 83.5 mg/kg | 35.25%* | 21.91% |
| *Malus niedzwetzkyana* Dieck: *Prunus cerasifera* | 25.96%** | 42.49% |
| Ehrhart 1:2 40 g/kg | | |
| *Malus niedzwetzkyana* Dieck: *Prunus cerasifera* | 35.58%* | 21.18% |
| Ehrhart 1:2 10 g/kg | | |
| *Malus niedzwetzkyana* Dieck: *Prunus cerasifera* | 17.02%** | 62.30% |
| Ehrhart 1:2 40 g/kg + aspirin 16.7 mg/kg | | |
| *Malus niedzwetzkyana* Dieck: *Prunus cerasifera* | 22.32%** | 50.55% |
| Ehrhart 1:2 10 g/kg + aspirin 16.7 mg/kg | | |
| *Malus niedzwetzkyana* Dieck: *Prunus cerasifera* | 17.43%** | 61.39% |
| Ehrhart 1:2 40 g/kg + clopidogrel 12.5 mg/kg | | |
| *Malus niedzwetzkyana* Dieck: *Prunus cerasifera* | 23.59%** | 47.74% |
| Ehrhart 1:2 10 g/kg + clopidogrel 12.5 mg/kg | | |
| *Malus niedzwetzkyana* Dieck: *Prunus cerasifera* | 19.27%** | 57.31% |
| Ehrhart 1:2 40 g/kg + colchicine 0.167 mg/kg | | |
| *Malus niedzwetzkyana* Dieck: *Prunus cerasifera* | 20.43%** | 54.74% |
| Ehrhart 1:2 40 g/kg + probucol 83.5 mg/kg | | |

| | | |
|---|---|---|
| The significance in the above table was in comparison with the model group; * p< 0.05, ** p<0.01, *** p<0.001 | | |

### Experimental example 7: Difference in effects of different varieties of Malus niedzwetzkyana Dieck and Prunus cerasifera Ehrhart in animal model

### (1) Test method

Wild type C57 mice were used as a normal control group, rosuvastatin was used as a positive drug, and the efficacy (i.e., treating effect) of the samples in resisting atherosclerosis was investigated in a high-fat diet induced ApoE-/-mice atherosclerosis model. An atherosclerosis model was established after 17 weeks of modeling, and a control group was fed with a common feed for 17 weeks. The sample administration group was continuously administered with the test samples for 4 weeks after 17 weeks of modeling, and thereafter the formation of aortic atherosclerotic plaque in the mice was observed, and the plaque area was measured after dissection to calculate the plaque percentage.

### (2) Test results

As could be seen from Table 7, the inhibition rates of *Malus niedzwetzkyana* Dieck Hongxun 1 and *Malus niedzwetzkyana* Dieck Hongxun 3 were similar, and there was no significant difference. It could be seen that there was no significant difference in the effects of different varieties of *Malus niedzwetzkyana* Dieck in animal model. Similarly, there was no significant difference in the effects of different varieties of *Prunus cerasifera* Ehrhart in animal model.

**Table 7 Effects of different varieties of Malus niedzwetzkyana Dieck for treating atherosclerosis in mice**

| Group | Plaque area % | Inhibition rate compared to the model % |
|---|---|---|
| Model | 39.66% | / |
| Positive drug (rosuvastain) 1.67 mg/kg | 25.87%** | 34.77% |
| *Malus niedzwetzkyana* Dieck (Hongxun 1) 20 g/kg | 32.89%* | 17.07% |
| *Malus niedzwetzkyana* Dieck (Hongxun 3) 20 g/kg | 32.56%* | 17.90% |

| | | |
|---|---|---|
| The significance in the above table was in comparison with the model group; * p< 0.05, ** p<0.01, *** p<0.001 | | |

Experimental example 8: Difference in effects of compositions of *Malus niedzwetzkyana* Dieck and *Prunus cerasifera* Ehrhart obtained by different extraction methods in animal model

### (1) Test method

Wild type C57 mice were used as a normal control group, rosuvastatin was used as a positive drug, and the efficacy (i.e., treating effect) of the composition samples in resisting atherosclerosis was investigated in a high-fat diet induced ApoE-/-mice atherosclerosis model. An atherosclerosis model was established after 17 weeks of modeling, and a control group was fed with a common feed for 17 weeks. The sample administration group was continuously administered with the test samples for 4 weeks after 17 weeks of modeling, and thereafter the formation of aortic atherosclerotic plaque in the mice was observed, and the plaque area was measured after dissection to calculate the plaque percentage.

### (2) Test results

As could be seen from Table 8, the compositions obtained according to Example 7 and Example 8 by extraction with different concentrations of ethanol each had a good effect in terms of treating atherosclerosis. It could be seen that the compositions of *Malus niedzwetzkyana* Dieck and *Prunus cerasifera* Ehrhart obtained by different extraction methods each had a good effect in animal model, being superior to that of the two used alone.

**Table 8 Effects of compositions of Malus niedzwetzkyana Dieck and Prunus cerasifera Ehrhart obtained by different extraction methods for treating atherosclerosis in mice**

| Group | Extraction method | Plaque area % | Inhibition rate compared to the model % |
|---|---|---|---|
| Model | / | 42.57% | / |
| Positive drug (rosuvastain) 1.67 mg/kg | / | 26.10%* | 38.69% |
| *Malus niedzwetzkyana* Dieck: *Prunus cerasifera* Ehrhart 1:2 40 g/kg | Leaching extraction with water according to Example 7 | 20.88%** | 50.95% |
| *Malus niedzwetzkyana* Dieck: *Prunus cerasifera* Ehrhart 1:2 40 g/kg | Leaching extraction with 20% ethanol according to Example 8 | 20.14%** | 52.69% |
| *Malus niedzwetzkyana* Dieck: *Prunus cerasifera* Ehrhart 1:2 40 g/kg | Leaching extraction with 40% ethanol according to Example 8 | 20.65%** | 51.49% |
| *Malus niedzwetzkyana* Dieck: *Prunus cerasifera* Ehrhart 1:2 40 g/kg | Leaching extraction with 60% ethanol according to Example 8 | 23.13%** | 45.67% |
| *Malus niedzwetzkyana* Dieck: *Prunus cerasifera* Ehrhart 1:2 40g/kg | Leaching extraction with 100% ethanol according to Example 8 | 25.96%* | 39.01% |

| | | | |
|---|---|---|---|
| The significance in the above table was in comparison with the model group; * p< 0.05, ** p<0.01, *** p<0.001 | | | |

Experimental example 9: Difference in effects of compositions of *Malus niedzwetzkyana* Dieck and *Prunus cerasifera* Ehrhart obtained by different extraction methods in animal model

### (1) Test method

Wild type C57 mice were used as a normal control group, rosuvastatin was used as a positive drug, and the efficacy (i.e., treating effect) of the composition samples in resisting atherosclerosis was investigated in a high-fat diet induced ApoE-/-mice atherosclerosis model. An atherosclerosis model was established after 17 weeks of modeling, and a control group was fed with a common feed for 17 weeks. The sample administration group was continuously administered with the test samples for 4 weeks after 17 weeks of modeling, and thereafter the formation of aortic atherosclerotic plaque in the mice was observed, and the mice were dissected, to calculate the plaque area (percentage).

### (2) Test results

As could be seen from Table 9, the compositions obtained according to Example 22 and Example 23 by gradient elution of macroporous adsorbent resin with different concentrations of ethanol each had a good effect in terms of treating atherosclerosis. It could be seen that the compositions of *Malus niedzwetzkyana* Dieck and *Prunus cerasifera* Ehrhart obtained by different extraction methods each had a good effect in animal model, being superior to that of the two used alone.

**Table 9 Effects of compositions of Malus niedzwetzkyana Dieck and Prunus cerasifera Ehrhart obtained by different extraction methods for treating atherosclerosis in mice**

| Group | Extraction method | Plaque area % | Inhibition rate compared to the model % |
|---|---|---|---|
| Model | / | 48.76% | / |
| Positive drug (rosuvastain) 1.67 mg/kg | / | 27.94%** | 42.69% |
| *Malus niedzwetzkyana* Dieck: *Prunus cerasifera* Ehrhart 1:2 40 g/kg | Clear alcohol precipitation juice according to Example 22 | 26.17%** | 46.33% |
| *Malus niedzwetzkyana* Dieck: *Prunus cerasifera* Ehrhart 1:2 40 g/kg | Elution of macroporous adsorbent resin with water according to Example 23 | 25.08%** | 48.56% |
| *Malus niedzwetzkyana* Dieck: *Prunus cerasifera* Ehrhart 1:2 40 g/kg | Gradient elution of macroporous adsorbent resin with 20% ethanol according to Example 23 | 23.79%** | 51.21% |
| *Malus niedzwetzkyana* Dieck: *Prunus cerasifera* Ehrhart 1:2 40 g/kg | Gradient elution of macroporous adsorbent resin with 40% ethanol according to Example 23 | 23.67%** | 51.45% |
| *Malus niedzwetzkyana* Dieck: *Prunus cerasifera* Ehrhart 1:2 40 g/kg | Gradient elution of macroporous adsorbent resin with 60% ethanol according to Example 23 | 27.16%** | 44.30% |
| *Malus niedzwetzkyana* Dieck: *Prunus cerasifera* Ehrhart 1:2 40g/kg | Gradient elution of macroporous adsorbent resin with 100% ethanol according to Example 23 | 29.20%* | 40.12% |

| | | | |
|---|---|---|---|
| The significance in the above table was in comparison with the model group; * p< 0.05, ** p<0.01, *** p<0.001 | | | |

The above examples are merely illustrative of the technical solutions of the invention, and not restrictive, and modifications and equivalent substitutions made thereto, without departing from the spirit and scope of the technical solutions of the invention, are intended to be included within the scope of the invention.

## Claims

1. A composition comprising:
a first component which is *Malus niedzwetzkyana* Dieck or *Malus niedzwetzkyana* Dieck extract; and
a second component which is *Prunus cerasifera* Ehrhart or *Prunus cerasifera* Ehrhart extract.

2. The composition of claim 1, wherein the weight ratio of the first component to the second component is 1:10 to 10:1 (such as 1:9-10:1, 1:8-10:1, 1:7-10:1, 1:6-10:1, 1:5-10:1, 1:4-10:1, 1:3-10:1, 1:2-10:1, 1:1-10:1, 1:10-9:1, 1:9-9:1, 1:8-9:1, 1:7-9:1, 1:6-9:1, 1:5-9:1, 1:4-9:1, 1:3-9:1, 1:2-9:1, 1:1-9:1, 1:10-8:1, 1:9-8:1, 1:8-8:1, 1-7-8-1, 1:6-8:1, 1:5-8:1, 1:4-8:1, 1:3-8:1, 1:2-8:1, 1:1-8:1, 1:10-7:1, 1:9-7:1, 1:8-7:1, 1:7-7:1, 1:6-7:1, 1:5-7:1, 1:4-7:1, 1:3-7:1, 1:2-7:1, 1:1-7:1, 1:10-6:1, 1:9-6:1, 1:8-6:1, 1:7-6:1, 1:6-6:1, 1:5-6:1, 1:4-6:1, 1:3-6:1, 1:2-6:1, 1:1-6:1, 1:10-5:1, 1:9-5:1, 1:8-5:1, 1:7-5:1, 1:6-5:1, 1:5-5:1, 1:4-5:1, 1:3-5:1, 1:2-5:1, 1:1-5:1, 1:10-4:1, 1:9-4:1, 1:8-4:1, 1:7-4:1, 1:6-4:1, 1:5-4:1, 1:4-4:1, 1:3-4:1, 1:2-4:1, 1:1-4:1, 1:10-3:1, 1:9-3:1, 1:8-3:1, 1:7-3:1, 1:6-3:1, 1:5-3:1, 1:4-3:1, 1:3-3:1, 1:2-3:1, 1:1-3:1, 1:10-2:1, 1:9-2:1, 1:8-2:1, 1:7-2:1, 1:6-2:1, 1:5-2:1, 1:4-2:1, 1:3-2:1, 1:2-2:1, 1:1-2:1, 1:10-1:1, 1:9-1:1, 1:8-1:1, 1:7-1:1, 1:6-1:1, 1:5-1:1, 1:4-1:1, 1:3-1:1, 1:2-1:1, 1:10-5:1, 1:10-2:1, 1:10-1:1, 1:5-10:1, 1:5-5:1, 1:5-2:1, 1:5-1:1, 1:2-10:1, 1:2-5:1, 1:2-2:1, 1:2-1:1, 1:1-10:1, 1:1-5:1, 1:1-2:1, e.g., 1:9, 1:8, 1:7, 1:6, 1:5, 1:4, 1:3, 1:2, 1:1, 2:1, 3:1, 4:1, 5:1, 6:1, 7:1, 8:1, 9:1).

3. The composition of any one of claims 1-2, wherein the *Malus niedzwetzkyana* Dieck or *Malus niedzwetzkyana* Dieck extract is obtained from/by: fresh fruit, fresh fruit pulping or juice extraction, leaching extraction, ultrasonic extraction, ultra-high pressure extraction, subcritical extraction, macroporous resin separation or other extraction methods;
or, the *Prunus cerasifera* Ehrhart or *Prunus cerasifera* Ehrhart extract is obtained from/by: fresh fruit, fresh fruit pulping or juice extraction, leaching extraction, ultrasonic extraction, ultra-high pressure extraction, subcritical extraction, macroporous resin separation or other extraction methods.

4. The composition of any one of claims 1-3, wherein the composition is in the form of fresh fruit, juice, jam, powder, candy, oral liquid, capsule, tablet, dried fruit, preserved fruit, wine, or pastry;
optionally, the forms including juice, jam, powder, candy, oral liquid, capsule, tablet and the like can be prepared using, as raw material, *Malus niedzwetzkyana* Dieck and *Prunus cerasifera* Ehrhart extracts obtained by leaching extraction, ultrasonic extraction, ultra-high pressure extraction, subcritical extraction, macroporous resin separation or other extraction methods;
or, the composition is in the form of a drug (e.g., traditional Chinese medicine, Chinese patent medicine, natural medicine and the like), ordinary food or functional food or health food.

5. The composition of any one of claims 1-4, wherein the *Malus niedzwetzkyana* Dieck refers to a fruit of a plant of Malus Mill of Rosaceae with red flesh, preferably, the *Malus niedzwetzkyana* Dieck is selected from: Hongxun 1 (Hong Xun 1), Hongxun 3 (Hong Xun 3), Hongxun 4 (Hong Xun 4), Hongxun 6 (Hong Xun 6), Hongxun 8 (Hong Xun 8), Red Love and the like;
or, the *Prunus cerasifera* Ehrhart is selected from *Prunus cerasifera* Ehrhart red fruit, *Prunus cerasifera* Ehrhart black fruit, *Prunus cerasifera* Ehrhart purple fruit, *Prunus cerasifera* Ehrhart purple black fruit, *Prunus cerasifera* Ehrhart yellow fruit, and the like.

6. A combination product comprising:
a first product, said first product being the composition of any one of claims 1-5;
a second product, said second product being a drug for preventing and/or treating atherosclerotic disease.

7. The combination product of claim 6, wherein the atherosclerotic disease is selected from atherosclerosis of aorta and its major branches, coronary atherosclerosis, cerebral atherosclerosis, renal atherosclerosis, mesenteric atherosclerosis, extremity atherosclerosis and the like;
or, the drug for preventing and/or treating atherosclerotic disease is selected from statins (e.g., rosuvastatin, atorvastatin, simvastatin), ezetimibe, PCSK9 inhibitor, aspirin, clopidogrel, colchicine, connamab, probucol, idebenone and the like, and Chinese patent medicine preparations.

8. The combination product of any one of claims 6-7, wherein the weight ratio of the first product to the second product is 1000:0.001 to 1000:33.4.

9. Use of the composition of any one of claims 1-5 in the preparation of medicine (e.g., traditional Chinese medicine, Chinese patent medicine, natural medicine and the like), ordinary food or functional food or health food for preventing and/or treating atherosclerotic disease.

10. Use of the combination product of any one of claims 6-8 in the preparation of medicine (e.g., traditional Chinese medicine, Chinese patent medicine, natural medicine and the like) for preventing and/or treating atherosclerotic disease.

11. The use of claim 9 or 10, wherein the atherosclerotic disease is selected from atherosclerosis of aorta and its major branches, coronary atherosclerosis, cerebral atherosclerosis, renal atherosclerosis, mesenteric atherosclerosis, extremity atherosclerosis and the like.
